# FASCICULE DE BREVET EUROPEEN

(11) **EP 0 765 668 B1**
(45) Date de publication et mention de la délivrance du brevet: **17.03.1999**
(21) Numéro de dépôt: 96401782.6
(22) Date de dépôt: 13.08.1996
(51) Int. Cl.: A61K 35/78, A61K 7/48, A61K 7/06, A61K 38/22, A61K 31/41, A61K 31/495

(54) **Extrait d'iridacees et compositions le contenant**
Extrakt von Iridaceen und Zusammensetzungen, die ihn enthalten
Extract of Iridaceas and compositions containing it

(30) Priorité: 07.09.1995 FR 9510486; 07.09.1995 FR 9510487; 27.03.1996 FR 9603815
(43) Date de publication de la demande: 02.04.1997
(73) Titulaire: L'OREAL, 75008 Paris (FR)
(72) Inventeur: Breton, Lionel, 78000 Versailles (FR); Martin, Richard, 37210 Rochecorbon (FR); De Lacharriere, Olivier, 75015 Paris (FR)
(74) Mandataire: Andral, Christophe André Louis

(56) Documents cités:
- EP-A- 0 680 749
- EP-A- 0 723 774
- WO-A-95/07294
- WO-A-95/35304
- FR-A- 2 728 793
- US-A- 5 364 881
- DATABASE WPI Section Ch, Week 9601 Derwent Publications Ltd., London, GB; Class D21, AN 96-006882 XP002004988 & JP-A-07 285 845 (POLA CHEM IND INC) , 31 Octobre 1995
- DATABASE WPI Section Ch, Week 9536 Derwent Publications Ltd., London, GB; Class B02, AN 95-272884 XP002004989 & JP-A-07 173 148 (KIKKOMAN CORP) , 11 Juillet 1995
- PATENT ABSTRACTS OF JAPAN vol. 012, no. 181 (C-499), 27 Mai 1988 & JP-A-62 289509 (SHISEIDO CO LTD), 16 Décembre 1987,
- PATENT ABSTRACTS OF JAPAN vol. 011, no. 259 (C-441), 21 Août 1987 & JP-A-62 061924 (SHISEIDO CO LTD), 18 Mars 1987,
- PATENT ABSTRACTS OF JAPAN vol. 95, no. 001 & JP-A-07 025762 (SANSHO SEIYAKU CO LTD), 27 Janvier 1995,
- GABRIEL GARNIER ET AL.: "RESSOURCES MEDICINALES DE LA FLORE FRANCAISE" 1961 , VIGOT FRERES , PARIS XP002004987 * page 277 - page 285 *

## Description

La présente invention a pour objet des compositions contenant un extrait de cellules d'au moins un végétal de la famille des Iridacées, l'utilisation desdits extraits comme antagoniste du CGRP et/ou de la substance P et un procédé de traitement cosmétique faisant intervenir une desdites compositions.

A la connaissance de la demanderesse des extraits de cellules d'au moins un végétal de la famille des Iridacées sont décrits dans l'art antérieur pour des propriétés qui ne sont pas celles mises en évidence par la demanderesse.
En effet, Garnier dans "Ressources médicinales de la flore française", (1961, Vigot Frères, pages 277-285) décrit un extrait de safran comme emménagogue et comme antispasmodique.
La demande de brevet JP-A-07173148 décrit des extraits d'Iridacées contenant de la génistein, composé ayant des activité oestrogénique, antibactérienne, antioxydante et/ou antinéoplasique.
La demande de brevet japonais JP-A-62289509 décrit des compositions cosmétiques destinées à faciliter le coiffage des cheveux, contenant au moins un extrait d'Iridacées.
La demande de brevet japonais JP-A- JP-A-62061924 décrit des compositions d'application topique contenant un extrait d'Iridacée, composition destinée à combattre la peau rugueuse.

La demanderesse ayant mis en évidence après de long travaux des propriétés particulières de l'extrait d'Iridacées, (antagoniste de substance P, antagoniste de CGRP), elle propose l'utilisation d'au moins un extrait de cellules d'au moins un végétal de la famille des Iridacées en tant qu'actif antagoniste de substance P et/ou antagoniste de CGRP dans des compositions cosmétique ou pour la préparation de compositions pharmaceutiques.

Par la suite le terme "cellules d'Iridacées" doit s'entendre comme "cellules d'au moins un végétal de la famille des Iridacées". De même le terme "extrait d'Iridacées" doit s'entendre comme "extrait de cellules d'Iridacées" et donc comme "extrait de cellules d'au moins un végétal de la famille des Iridacées".

L'extrait de cellules d'au moins un végétal de la famille des Iridacées, peut être un extrait préparé à partir de tout matériel végétal issu de la famille des Iridacées, ledit matériel ayant été obtenu par culture *in vitro*.

La pression de sélection imposée par les conditions physico-chimiques lors de la croissance des cellules végétales *in vitro* permet d'obtenir un matériel végétal standardisé et disponible tout au long de l'année contrairement aux plantes obtenu par culture *in vivo*.

Par culture *in vitro*, on entend l'ensemble des techniques connues de l'homme du métier qui permet de manière artificielle l'obtention d'un végétal ou d'une partie d'un végétal.
Ainsi, par exemple selon l'invention l'extrait peut être un extrait d'organe voire de cellules d'organe d'au moins une Iridacée obtenu par culture *in vitro* (racines, tige, feuille) ou encore un extrait de cellules indifférenciées d'au moins une Iridacée.

De préférence on utilise un extrait obtenu à partir de cellules indifférenciées obtenues par culture *in vitro.*

Par cellules végétales indifférenciées, on entend toute cellule végétale ne présentant aucun des caractères d'une spécialisation particulière et capable de vivre par elle-même et non en dépendance avec d'autres cellules. Ces cellules végétales indifférenciées sont éventuellement aptes, sous l'effet d'une induction, à toute différenciation conforme à leur génome.
Selon la méthode de culture choisie, et en particulier selon le milieu de culture choisi, il est possible d'obtenir à partir d'un même explant des cellules végétales indifférenciées présentant des caractères différents.

La famille des Iridacées (ou Iridées) compte environ 750 espèces.
Les plantes de la famille des Iridacées sont surtout utilisées pour leur propriétés aromatiques et ornementales.
Parmi les genres d'Iridacées utilisables selon l'invention, on peut citer à titre d'exemple les genres Romulea, Crocus, Iris, Gladiolus, Sisyrinchium ou encore Hermodactylus.
Comme matériel végétal utilisable on peut citer celui provenant *d'Iris germanica, d'Iris florentina, d'Iris pallida,* de *Crocus versicolor,* de *Romulea bulbucodium* ou encore de *Gladiolus communis.*

Plus particulièrement selon l'invention on utilise du matériel végétal issu du genre Iris, plus particulièrement *d'Iris pallida.*

Toute méthode d'extraction connue de l'homme du métier peut être utilisée selon l'invention.
On peut en particulier citer les extraits alcooliques, notamment éthanoliques, les extraits hydroalcooliques.

On peut également utiliser un extrait préparé par la méthode décrite dans la demande de brevet français n° 95-02379.
Ainsi, dans une première étape on broie le matériel végétal dans une solution aqueuse à froid, dans une deuxième étape les particules en suspension sont éliminées de la solution aqueuse issue de la première étape, et dans une troisième étape on stérilise la solution aqueuse issue de la deuxième étape. Cette solution aqueuse correspond à l'extrait.
D'autre part, la première étape peut avantageusement être remplacée par une opération de congélation simple des tissus végétaux (par exemple à -20°C), suivie d'une extraction aqueuse reprenant les deuxièmes et troisième étapes ci-dessus décrites.
Un exemple de préparation d'extrait utilisable selon l'invention est donné par ailleurs dans les exemples.

L'invention a également pour objet une composition cosmétique ou pharmaceutique comprenant à titre de principe actif dans un milieu cosmétiquement ou pharmaceutiquement acceptable, au moins un extrait de matériel végétal d'au moins une Iridacée tel que défini précédement.

Par principe actif, on entend toute molécule ou extrait susceptible de modifier ou de moduler le fonctionnement d'au moins un système biologique donné.

Il existe chez les mammifères des polypeptides appartenant à la famille des tachykinines qui induisent sur les fibres musculaires lisses des contractions rapides. Parmi les composés de cette famille, on peut citer la neurokinine β, neurokinine α et la substance P.

La substance P est un élément chimique polypeptidique (undécapeptide), élaboré et libéré par une terminaison nerveuse. La localisation de la substance P est spécifiq,ue des neurones, tant dans le système nerveux central que dans les organes à la périphérie. Ainsi, de très nombreux organes ou tissus reçoivent des afférences de neurones à substance P, il s'agit notamment des glandes salivaires, de l'estomac, du pancréas, de l'intestin (dans celui-ci, la distribution de la substance P est superposée au plexus nerveux intrinsèque de Meissner et d'Auerbach), du système cardio-vasculaire, de la glande thyroïde, de la peau, de l'iris et des corps ciliaires, de la vessie et bien évidemment des système nerveux central et périphérique.

De par la distribution ubiquitaire de la substance P, de très nombreux désordres sont associés à un excès de synthèse et / ou de libération de substance P.

La substance P intervient notamment dans la transmission de la douleur et dans des maladies du système nerveux central (par exemple l'anxiété, les psychoses, les neuropathies, les troubles neurodégénératifs de type démence sénile d'Alzheimer, démence des sidéens, maladie de Parkinson, syndrome de Down, syndrome de Korsakoff, scléroses multiples, schizophrénie), dans des maladies respiratoires (telles que par exemple les broncho-pneumonie) et inflammatoires (telles que par exemple la polyarthrite rhumatoïde), dans des syndromes allergiques (tels que par exemple l'asthme, les rhinites allergiques, les pharyngites allergiques, l'urticaire, les dermatites eczémateuses), dans des maladies gastro-intestinales (telles que par exemple les ulcères, les colites, la maladie de Crohn), dans des désordres cutanés (tels que par exemple le psoriasis, les maladies prurigineuses, l'herpès, les photodermatoses, les dermatites atopiques, les dermatites de contact, les lichens, les prurigos, les prurits, les érythèmes en particulier solaire, les piqûres d'insectes), dans des fibroses et autres troubles de la maturation des collagènes (tels que par exemple la sclérodermie), dans des troubles cardio-vasculaires, des troubles vasospastiques (tels que par exemple les migraines, la maladie de Reynaud), dans des désordres immunologiques, dans des troubles du tractus urinaire (tels que par exemple l'incontinence, la cystite), dans des maladies rhumatismales, dans certaines maladies dermatologiques (telles que l'eczéma) et dans les affections ophtalmologiques (telles que par exemple les conjonctivites, les uvéites, les prurits oculaires, les douleurs oculaires, les irritations ).

L'utilisation d'antagoniste de substance P est l'une des alternatives thérapeutiques efficaces dans toutes les affections citées ci-dessus.

Par antagoniste de substance P, on entend tout composé susceptible d'inhiber partiellement, voire totalement, l'effet biologique de la substance P.
Particulièrement, pour qu'une substance soit reconnue comme un antagoniste de substance P elle doit induire une réponse pharmacologique cohérente (incluant ou non sa fixation au récepteur de la substance P) notamment dans l'un des tests suivants :
- la substance antagoniste doit diminuer l'extravasation du plasma au travers de la paroi vasculaire induite par la capsaïcine ou par une stimulation nerveuse antidromique, ou bien
- la substance antagoniste doit provoquer une inhibition de la contraction des muscles lisses induites par l'administration de substance P.

A ce jour, des antagonistes de substance P sont utilisés pour traiter les désordres indiqués ci-dessus. A cet effet, on peut se référer aux documents US-A-4472305, US-A-4839465, EP-A-101929, EP-A-333174, EP-A-336230, EP-A-394989, EP-A-443132, EP-A-498069, EP-A-515681, EP-A-517589, WO-A-92/22569, GB-A-2216529, EP-A-360390, EP-A-429366, EP-A-430771, EP-A-499313, EP-A-514273, EP-A-514274, EP-A-514275, EP-A-514276, EP-A-520555, EP-A-528495, EP-A-532456, EP-A-545478, EP-A-558156, WO-A-90/05525, WO-A-90/05729, WO-A-91/18878, WO-A-91/18899, WO-A-92/12151, WO-A-92/15585, WO-A-92/17449, WO-A-92/20676, WO-A-93/00330, WO-A-93/00331, WO-A-93/01159, WO-A-93/01169, W0-A-93/01170, WO-A-93/06099, WO-A-93/09116, EP-A-522808 et WO-A-93/01165.

Cependant aucun des documents ci-dessus n'envisage, ni ne suggère qu'un extrait d'au moins une Iridacée puisse avoir une activité antagoniste de substance P telle que définie ci-dessus et donc puisse être notamment utilisé pour traiter les désordres indiqués ci-dessus.

La demanderesse a découvert qu'un extrait d'au moins une Iridacée répond aux caractéristiques définies comme caractérisant un antagoniste de substance P et peut donc être utilisé comme antagoniste de substance P.

Le CGRP est un élément chimique polypeptidique élaboré et libéré par une terminaison nerveuse.
La localisation du CGRP est spécifique des fibres nerveuses sensitives (fibres C). Ainsi, de très nombreux organes ou tissus reçoivent des afférences de neurones à CGRP, il s'agit notamment des glandes salivaires, de l'estomac, du pancréas, de l'intestin, du système cardio-vasculaire, de la glande thyroïde et de la peau.

De par la distribution ubiquitaire du CGRP, de très nombreuses pathologies sont associées à un excès de synthèse et / ou de libération du CGRP.
Il s'agit notamment de maladies respiratoires et inflammatoires, des maladies allergiques et de désordres cutanés, notamment d'affections dermatologiques telles que l'eczéma, le prurigo, la rosacée.

L'utilisation d'antagoniste du CGRP est l'un des traitements alternatifs efficaces dans toutes les affections citées ci-dessus.

Par antagoniste du CGRP, on entend tout composé susceptible d'inhiber partiellement, voire totalement, l'effet biologique du CGRP.
Particulièrement, pour qu'une substance soit reconnue comme un antagoniste de CGRP elle doit induire une réponse pharmacologique cohérente (incluant ou non sa fixation au récepteur du CGRP) notamment dans l'un des tests suivants :
+ la substance antagoniste doit diminuer la vasodilatation induite par la capsaïcine et/ou par une stimulation électrique antidromique (appliquée sur un nerf afférent) et/ou
+ la substance antagoniste doit provoquer une inhibition de la libération de CGRP par les fibres nerveuses sensitives et/ou
+ la substance antagoniste doit provoquer une inhibition de la contraction du muscle lisse du vas deferens induite par le CGRP.

Parmi les antagonistes du CGRP connus, on peut citer par exemple le CGRP 8-37 (séquence des acides aminés 8 à 37 de la partie N-terminale du CGRP), ou encore les anticorps anti-CGRP.

A ce jour, il n'a pas été envisagé ni même suggéré qu'un extrait d'au moins une Iridacée puisse avoir une activité antagoniste du CGRP.

La demanderesse a découvert qu'un extrait d'au moins une Iridacée répond aux caractéristiques définies comme caractérisant un antagoniste du CGRP et peut donc être utilisé comme antagoniste du CGRP.

On a vu par ailleurs dans le texte des exemples de désordres liés associés à un excès de synthèse et / ou de libération de substance P ou à un excès de synthèse et/ou de libération du CGRP.

Ainsi, l'invention a donc pour objet une composition cosmétique ou pharmaceutique comprenant à titre de principe actif dans un milieu cosmétiquement ou pharmaceutiquement acceptable, au moins un extrait de matériel végétal d'au moins une Iridacée destinée à traiter les désordres du système nerveux central, les troubles respiratoires, les syndromes allergiques, l'inflammation, la douleur, les désordres gastro-intestinaux, les désordres cutanés, les fibroses, les troubles de la maturation du collagène les troubles cardio-vasculaires, les troubles vasospastiques, les désordres immunologiques et/ou les troubles du tractus urinaires.

Dans le domaine des désordres cutanés, il est connu que certaines peaux sont plus sensibles que d'autres. Toutefois, les symptômes des peaux sensibles étaient jusqu'à présent mal caractérisés et le problème de ces peaux était, de ce fait, mal défini ; personne ne connaissait exactement le processus mis en cause dans la sensibilité de la peau. Certains pensaient qu'une peau sensible était une peau qui réagissait aux produits cosmétiques, d'autres qu'il s'agissait d'une peau qui réagissait à plusieurs facteurs extérieurs, pas forcément liés aux produits cosmétiques. On assimilait également les peaux sensibles à des peaux allergiques.

Des tests ont été mis au point pour cerner les peaux sensibles, par exemple des tests à l'acide lactique et au DMSO qui sont connus pour être des substances irritantes : voir par exemple l'article de K. Lammintausta et coll., Dermatoses, 1988, 36, pages 45-49; et l'article de T. Agner et J. Serup, Clinical and Experimental Dermatology, 1989, 14, pages 214-217.

Du fait de la méconnaissance des caractéristiques des peaux sensibles, il était jusqu'à présent très difficile voire impossible de les traiter. En fait, on les traitait indirectement, par exemple en limitant dans les compositions cosmétiques ou dermatologiques l'emploi de produits à caractère irritant tels que les tensioactifs, les conservateurs ou les parfums ainsi que l'emploi de certains actifs cosmétiques ou dermatologiques.

Après de nombreux tests cliniques, la demanderesse a pu déterminer les symptômes liés aux peaux sensibles. Ces symptômes sont en particulier des signes subjectifs, qui sont essentiellement des sensations dysesthésiques. On entend par sensations dysesthésiques des sensations plus ou moins douloureuses ressenties dans une zone cutanée comme les picotements, fourmillements, démangeaisons ou prurits, brûlures, échauffements, inconforts, tiraillements. Les symptômes liés à la peau peuvent être également des manifestations microvasculaires du tissus cutané comme les érythèmes.

La demanderesse a pu montrer en outre qu'une peau sensible n'était pas une peau allergique. En effet, une peau allergique est une peau qui réagit à un agent extérieur, un allergène, qui déclenche une réaction d'allergie. Il s'agit d'un processus immunologique qui ne se produit que lorsqu'un allergène est présent et qui ne touche que les sujets sensibilisés. La caractéristique essentielle de la peau sensible est selon la demanderesse, au contraire, un mécanisme de réponse à des facteurs extérieurs, qui peut concerner tout individu, même si les individus dits à peau sensible y réagissent plus vite que les autres. Ce mécanisme n'est pas immunologique, il est aspécifique.

La demanderesse a trouvé que les peaux sensibles pouvaient être scindées en deux grandes formes cliniques, les peaux irritables et/ou réactives, et les peaux intolérantes.

Une peau irritable et/ou réactive est une peau qui réagit par un prurit, c'est-à-dire par des démangeaisons, ou par des picotements, à différents facteurs tels que l'environnement, les émotions, les aliments, le vent, les frottements, le rasoir, le savon, les tensioactifs, l'eau dure à forte concentration de calcaire, les variations de température ou la laine. En général, ces signes sont associés à une peau sèche avec ou sans dartres, ou à une peau qui présente un érythème.

Une peau intolérante est une peau qui réagit par des sensations d'échauffement, de tiraillements, de fourmillements et/ou de rougeurs, à différents facteurs tels que l'environnement, les émotions, les aliments et certains produits cosmétiques. En général, ces signes sont également associés à une peau hyperséborrhéique ou acnéique avec ou sans dartres, et à un érythème.

Les cuirs chevelus "sensibles" ont une sémiologie clinique plus univoque : les sensations de prurit et/ou de picotements et/ou d'échauffements sont essentiellement déclenchés par des facteurs locaux tels que frottements, savon, tensioactifs, eau dure à forte concentration de calcaire, shampooings ou lotions. Ces sensations sont aussi parfois déclenchées par des facteurs tels que l'environnement, les émotions et/ou les aliments. Un érythème et une hyperséborrhée du cuir chevelu ainsi qu'un état pelliculaire sont fréquemment associés aux signes précédents.

Par ailleurs, dans certaines régions anatomiques comme les grands plis (régions inguinales, génitale, axillaires, poplitées, anale, sous-mammaires, plis du coude) et les pieds, la peau sensible se traduit par des sensations prurigineuses et/ou des sensations dysesthésiques (échauffement, picotements) liées en particulier à la sueur, aux frottements, à la laine, aux tensioactifs, à certaines préparations cosmétiques, à l'eau dure à forte concentration en calcaire et/ou aux variations de température.

Pour déterminer si une peau est sensible ou non, la demanderesse a également mis au point un test. En effet, après avoir effectué un grand nombre de tests dans le but de définir une peau sensible, elle a trouvé de manière surprenante qu'il existait un lien entre les personnes à peau sensible et celles qui réagissaient à une application topique de capsaïcine.

Le test à la capsaïcine consiste à appliquer sur environ 4 cm² de peau 0,05 ml d'une crème comprenant 0,075 % de capsaïcine et à noter l'apparition de signes subjectifs provoqués par cette application, tels que picotements, brûlures et démangeaisons. Chez les sujets à peaux sensibles, ces signes apparaissent entre 3 et 20 minutes après l'application et sont suivis de l'apparition d'un érythème qui débute à la périphérie de la zone d'application.

Jusqu'à présent, la capsaïcine était utilisée comme médicament, en particulier pour traiter les douleurs du zona. La capsaïcine provoque un relargage des neuropeptides, et en particulier du CGRP et des tachykinines qui proviennent de terminaisons nerveuses de l'épiderme et du derme. La demanderesse a constaté que le schéma physiopathologique commun à tous les états des peaux sensibles était lié à une grande aptitude à libérer des neuropeptides et des tachykinines et plus particulièrement du CGRP et de la substance P dans la peau. Les manifestations dysesthésiques qui sont provoquées par leur libération sont dites "neurogènes".

Personne jusqu'à ce jour n'avait établi un lien entre le CGRP et la peau sensible et/ou la substance P et la peau sensible. Les signes cliniques de la peau sensible sont essentiellement subjectifs : picotements, fourmillements, prurits, tiraillements, échauffements, et ils s'associent parfois à des érythèmes. Ces signes sont dus à des facteurs extérieurs aspécifiques. Les symptômes apparaissent essentiellement localisés au visage, au cou et au cuir chevelu, mais peuvent apparaître aussi sur tout le corps.

Ainsi la demanderesse a découvert qu'une des caractéristiques essentielles des peaux sensibles est liée à la libération du CGRP et/ou de substance P et donc que l'utilisation d'antagonistes du CGRP et/ou d'antagoniste de substance P, dont en particulier un extrait de matériel végétal d'au moins une Iridacée, peut permettre d'obtenir un effet préventif et/ou curatif des peaux sensibles.

Ainsi, l'invention a pour objet une composition cosmétique ou pharmaceutique destinée à traiter les peaux sensibles, caractérisée en ce qu'elle comprend dans un milieu cosmétiquement ou pharmaceutiquement acceptable, à titre de principe actif, au moins un extrait de matériel végétal tel que défini précédement.

Pour traiter les peaux sensibles, la demanderesse a donc envisagé d'utiliser des antagonistes du CGRP et/ou des antagonistes de substance P. Elle a en effet constaté de manière surprenante que l'incorporation d'un antagoniste du CGRP et/ou un antagoniste de substance P dans une composition destinée à un usage topique permet d'éviter l'irritation cutanée et/ou les sensations dysesthésiques et/ou les prurits de la peau et/ou des muqueuses et/ou l'érythème et/ou les dartres et/ou les sensations d'échauffement.

L'invention a donc pour objet une composition cosmétique ou pharmaceutique destinée à traiter l'irritation cutanée et/ou les sensations dysesthésiques et/ou les prurits de la peau et/ou des muqueuses et/ou l'érythème et/ou les dartres et/ou les sensations d'échauffement.

De façon avantageuse, selon l'invention au moins un extrait d'au moins une Iridacée peut être associé à des produits à effet irritant utilisés couramment dans le domaine cosmétique ou pharmaceutique, produits qui sont parfois des actifs cosmétiques ou pharmaceutiques. La présence d'un antagoniste de substance P sous la forme d'au moins un extrait d'au moins une Iridacée dans une composition cosmétique ou pharmaceutique comprenant un produit ayant un effet irritant permet d'atténuer fortement, voire de supprimer cet effet irritant. Cela permet en outre d'augmenter la quantité de principe actif à effet irritant par rapport à la quantité de principe actif normalement utilisée, en vue d'une efficacité améliorée.

L'invention concerne plus particulièrement une composition cosmétique ou pharmaceutique, caractérisée en ce quelle comprend, dans un milieu cosmétiquement ou pharmaceutiquement acceptable, au moins un produit à effet irritant à l'exception de l'acide ascorbique et au moins un extrait d'au moins une Iridacée.

Comme produits à effet irritant, on peut citer par exemple les tensioactifs (ioniques ou non-ioniques), les conservateurs, les solvants organiques ou les actifs comme les α-hydroxy-acides (acide citrique, malique, glycolique, tartrique, mandélique, lactique), les β-hydroxyacides (l'acide salicylique et ses dérivés), les α-cétoacides, les β-cétoacides, les rétinoïdes (rétinol, rétinal, acide rétinoïque), les anthralines (dioxyanthranol), les anthranoïdes, les peroxydes (notamment de benzoyle), le minoxidil, les sels de lithium, les antimétabolites, la vitamine D et ses dérivés, les teintures ou colorants capillaires (paraphénylènediamine et ses dérivés, les aminophénols), les solutions alcooliques parfumantes (parfums, eaux de toilette, après rasage, déodorants), les agents antitranspirants (certains sels d'aluminium), les actifs dépilatoires ou de permanentes (thiols), les actifs dépigmentants (hydroquinone).

L'emploi d'antagoniste de substance P permet notamment de multiplier de 2 à 10 fois la quantité de principe actif à effet irritant par rapport à l'état de la technique, sans ressentir tous les inconforts mentionnés ci-dessus. Ainsi, on peut utiliser les hydroxyacides jusqu'à 50 % du poids de la composition ou les rétinoïdes jusqu'à 5 %, en diminuant notablement leur caractère irritant.

Préférentiellement, l'extrait d'au moins une Iridacée est un extrait comme décrit précédement dans le texte.

On sait par ailleurs qu'il existe au niveau cutané de nombreux phénomènes d'intolérance dont les symptômes sont en particulier des signes subjectifs, qui sont essentiellement des sensations dysesthésiques. On entend par sensations dysesthésiques des sensations plus ou moins douloureuses ressenties dans une zone cutanée comme les picotements, fourmillements, démangeaisons ou prurits, brûlures, échauffements, inconforts, tiraillements, etc.

Ces phénomènes peuvent être la conséquence d'événements multiples dont les plus banaux seront qualifiés d'irritation ou d'inflammation, mais dont certains seront dus à des causes physiologiques, comme les peaux sensibles, voire même pathologiques comme par exemple, l'allergie.

Mais, la peau sensible peut également réagir par des sensations d'échauffement, de tiraillements, de fourmillements et/ou de rougeurs, à différents facteurs tels que l'environnement, les émotions ou les aliments. En général, ces signes sont associés à une peau hyperséborrhéique ou acnéique, avec ou sans dartres. Là encore, ces signes sont souvent associés à un érythème.

Ces phénomènes peuvent être généralisés à l'ensemble du corps, mais la plupart du temps ils peuvent avoir des localisations bien définies telles que par exemple le cuir chevelu, le visage, les plis cutanés, etc.

L'ensemble de ces phénomènes d'intolérance est toujours lié à un processus inflammatoire classique, et plus particulièrement à une réaction inflammatoire de type neurogène puisqu'elle fait intervenir les fibres nerveuses cutanées.

Une peau allergique est une peau qui réagit à un agent extérieur, un allergène, qui déclenche une réaction d'allergie. Il s'agit alors d'un processus spécifiquement immunologique qui ne se produit que lorsqu'un allergène est présent et qui ne touche que les sujets sensibilisés. Par contre, la résultante finale d'une réaction allergique se traduit également par une réaction inflammatoire aiguë associé généralement à un oedème.

Quelque soit le phénomène envisagé, il existe un point commun à tous ces mécanismes qui se traduit par une réaction inflammatoire dont la facette terminale se mesure par la libération par les cellules mastocytaires de la peau d'au moins un médiateur de l'inflammation tels que l'histamine, la sérotonine, l'héparine, les leukotriènes, les prostaglandines, les cytokines, le monoxyde d'azote ou des espèces oxygénées réactives.

Dans certains cas, comme par exemple les peaux sensibles, l'ensemble du mécanisme est également sous le contrôle des terminaisons nerveuses sensitives qui libèrent des neuropeptides, notamment la substance P et le CGRP.

Le but recherché par la présente invention est d'obtenir un effet bénéfique le plus étendu possible dans le traitement de toutes ces affections cutanées et donc de proposer une composition qui agit sur plusieurs composantes de ces affections.

Ainsi, selon un autre aspect la présente invention a pour objet une composition cosmétique ou pharmaceutique, caractérisée en ce qu'elle comprend, dans un milieu cosmétiquement ou pharmaceutiquement acceptable, un extrait d'au moins une Iridacée et un composé diminuant la synthèse, la libération et/ou l'activité d'au moins un médiateur de l'inflammation, à l'exception de l'acide kojique.

Préférentiellement, l'extrait d'au moins une Iridacée est un extrait comme décrit précédement dans le texte.

Parmi les agents anti-inflammatoires stéroïdiens on peut citer à titre d'exemple l'hydrocortisone, le valérate de bétaméthasone ou le propionate de clobétasol.

Par agents anti-inflammatoires non-stéroïdiens, on entend ici les agents anti-inflammatoires tels que décrits par Schorderet et Dayer dans Pharmacologie, "Des concepts fondamentaux aux applications thérapeutiques", 1992, chapitre 37, pages 541-561, 2ième édition, Frison-Roche/Slatkine éditeurs. Il s'agit des acides arylcarboxyliques, comme les dérivés de l'acide salicylique ou les dérivés de l'acide anthranilique, des acides arylalcanoïques, tels que les acides arylacétiques et hétéro-arylacétiques ou les acides arylpropioniques, des acides énoliques, comme les dérivés de la pyrazolone ou les oxicames, et des dérivés non acides, comme par exemple le bufexamac (Merck Index, 11ième édition (M.I.) 1462), la benzydamine (M.I. 1136), l'épirizole (M.I. 3572), la fluproquazone (M.I. 4120) ou la tiaramide (M.I. 9356).

Préférentiellement, la composition selon l'invention comprend un composé diminuant la synthèse, la libération et/ou l'activité d'au moins un médiateur de l'inflammation cutanée en association avec l'extrait d'au moins une Iridacée.

Le composé diminuant la synthèse, la libération et/ou l'activité d'au moins un médiateur de l'inflammation est de préférence choisi parmi les antagonistes de substance P et/ou de CGRP, les inhibiteurs de NO-synthase, les antagonistes de bradykinine, les antagonistes de cytokines, les antagonistes d'histamine, les antagonistes du facteur de nécrose tumorale de type α (TNFα).

Préférentiellement, on utilise des antagonistes réceptoriels.

Par exemple, selon l'invention, on peut utiliser un ou plusieurs antagonistes de substance P choisis parmi les peptides, les composés non peptidiques comme ceux comprenant au moins un hétérocycle, les composés azotés comprenant au moins un cycle benzénique, les sels de cations monovalents, divalents et trivalents, les eaux thermales, et leurs mélanges.
On peut utiliser dans l'invention par exemple comme peptide antagoniste de substance P le sendide et le spantide II.

Le sendide correspond à la formule :
Tyr D-Phe Phe D-His Leu Met NH₂
dans laquelle :
Tyr représente la tyrosine,
D-Phe représente la D-phénylalanine,
Phe représente la phénylalanine,
D-His représente la D-histidine,
Leu représente la leucine,
Met représente la méthionine.

Le spantide II correspond à la formule :
D-NicLys Pro 3-Pal Pro D-Cl₂Phe Asn D-Trp Phe D-Trp Leu Nle NH₂
dans laquelle :
D-NicLys représente.le nicotinate de D-lysine,
Pro représente la proline,
3-Pal représente la 3-pyridyl-alanine,
D-Cl₂Phe représente la D-dichlorophénylalanine,
Asn représente l'asparagine,
D-Trp représente le D-tryptophane,
Phe représente la phénylalanine,
Leu représente la leucine,
Nle représente la nor-leucine.

On peut également utiliser dans l'invention, comme peptide antagoniste de substance P, les peptides décrits dans les documents US-A-4472305, US-A-4839465, EP-A-101929, EP-A-333174, EP-A-336230, EP-A-394989, EP-A-443132, EP-A-498069, EP-A-515681, EP-A-517589, WO-A-92/22569 et GB-A-2216529.

Les antagonistes de substance P non peptidiques utilisables dans l'invention sont notamment des composés comprenant un hétéroatome lié directement ou indirectement à un cycle benzénique ou contenu dans un hétérocycle. En particulier cet hétéroatome est un atome d'oxygène, d'azote ou de soufre.

Comme composé hétérocyclique, on peut notamment utiliser dans l'invention ceux décrits dans les documents suivants : EP-A-360390, EP-A-429366, EP-A-430771, EP-A-499313, EP-A-514273, EP-A-514274, EP-A-514275, EP-A-514276, EP-A-520555, EP-A-528495, EP-A-532456, EP-A-545478, EP-A-558156, WO-A-90/05525, WO-A-90/05729, WO-A-91/18878, WO-A-91/18899, WO-A-92/12151, WO-A-92/15585, WO-A-92/17449, WO-A-92/20676, WO-A-93/00330, WO-A-93/00331, WO-A-93/01159, WO-A-93/01169, WO-A-93/01170, WO-A-93/06099, WO-A-93/09116.

En particulier, le composé comprenant au moins un hétérocycle azoté est un dérivé de 2-tricyclyl-2-amino-éthane, un dérivé de spirolactame, un dérivé de quinuclidine, un dérivé azacyclique, un dérivé d'aminopyrrolidine, un dérivé de pipéridine, un aminoazahétérocycle ou un dérivé d'isoindole.

Comme autres composés hétérocycliques, on peut citer les composés hétérocycliques oxygénés ou soufrés tels que les dérivés du furanne, les dérivés du benzofuranne, les dérivés du thiophène et les dérivés du benzothiophène, comportant éventuellement des substituants azotés, tels que les composés hétérocycliques décrits dans les documents US-A-4931459, US-A-4910317 et EP-A-299457, et plus spécialement les alcoxy- et/ou aryloxy- tétrazolyl-benzofuranne-carboxamides ou les alcoxy- et/ou aryloxy- tétrazolyl-benzothiophène-carboxamides.

Comme composés comportant un atome d'azote lié directement ou indirectement à un noyau benzénique, on peut citer ceux décrits dans les documents suivants : EP-A-522808 et WO-A-93/01165 et WO-A-93/10073.

Les sels de cations utilisables dans l'invention sont notamment les sels de strontium, de magnésium, de lanthanides de numéro atomique allant de 57 à 71, de cobalt, de nickel, de manganèse, de baryum, d'yttrium, de cuivre, d'étain, de rubidium, de lithium et de zinc.

Ces sels peuvent être par exemple des carbonates, des salicylates, des bicarbonates, des sulfates, des glycérophosphates, des borates, des chlorures, des nitrates, des acétates, des hydroxydes, des persulfates ainsi que des sels d'α-hydroxyacides (citrates, tartrates, lactates, malates) ou d'acides de fruits, ou encore des sels d'acides aminés (aspartate, arginate, glycocholate, fumarate) ou des sels d'acides gras (palmitate, oléate, caséinate, béhénate). De préférence, le sel est choisi parmi le nitrate de strontium, de manganèse, d'yttrium ou de magnésium, le borate de strontium, de manganèse, d'yttrium ou de magnésium, le chlorure de strontium, de manganèse ou de magnésium, le sulfate de magnésium, de manganèse ou de strontium. Encore plus préférentiellement, ces sels sont le chlorure ou le nitrate de strontium.

Parmi les eaux thermales utilisables selon l'invention on peut citer plus particulièrement les eaux thermales du bassin de Vichy, comme celles provenant des sources Célestins, Chomel, Grande-Grille, Hôpital, Lucas et Parc. Préférentiellement, selon l'invention, on utilise l'eau de la source Lucas.

Les antagonistes de substance P peuvent être utilisés seuls ou en mélange.

Par antagoniste du CGRP, on entend tout composé susceptible d'inhiber partiellement, voire totalement, l'effet biologique du CGRP.
Particulièrement, pour qu'une substance soit reconnue comme un antagoniste de CGRP elle doit induire une réponse pharmacologique cohérente (incluant ou non sa fixation au récepteur du CGRP) notamment dans l'un des tests suivants :
+ la substance antagoniste doit diminuer la vasodilatation induite par la capsaïcine et/ou par une stimulation électrique antidromique (appliquée sur un nerf afférent) et/ou
+ la substance antagoniste doit provoquer une inhibition de la libération de CGRP par les fibres nerveuses sensitives et/ou
+ la substance antagoniste doit provoquer une inhibition de la contraction du muscle lisse du vas deferens induite par le CGRP.

Parmi les antagonistes du CGRP connus, on peut citer par exemple le CGRP 8-37 (séquence des acides aminés 8 à 37 de la partie N-terminale du CGRP), ou encore les anticorps anti-CGRP.

Les antagonistes de CGRP peuvent être utilisés seuls ou en mélange.

Le terme NO-synthase recouvre en fait une famille d'enzymes qui, de façon spécifique, assurent la catalyse enzymatique de la L-arginine en citrulline, catalyse au cours de laquelle est produit un médiateur gazeux aux multiples fonctions, le monoxyde d'azote ou NO. Le monoxyde d'azote possède de par sa structure un électron supplémentaire le rendant extrêmement réactif chimiquement. Il est notoire que de tels composés sont nocifs et l'on cherche à limiter au mieux leur production. C'est ainsi que dans le cas du monoxyde d'azote les inhibiteurs de NO-synthase ont été largement étudiés.

Ainsi, selon l'invention, les inhibiteurs de NO-synthase sont des produits qui permettent *in situ* sur l'homme d'inhiber partiellement, voire totalement, la synthèse de monoxyde d'azote (NO).
Ce sont donc des composés choisis parmi les composés inhibant la synthèse et/ou accélérant le catabolisme de la NO-synthase, les composés neutralisant la NO-synthase ou les composés intervenant en diminuant le signal transduit par la NO-synthase.

Ainsi, l'inhibiteur de la NO-synthase peut être choisi parmi des peptides, synthétiques ou naturels, éventuellement modifiés, des molécules chimiques, synthétiques ou naturelles, des acides nucléiques antisens, des ribozymes, des anticorps anti-NO-synthase.

Parmi ces inhibiteurs de la NO-synthase, on peut citer notamment la N^{G}-monométhyl-L-arginine (L-NMMA), la N^{G}-nitro-L-arginine, l'ester méthylé de la N^{G}-nitro-L-arginine, le chlorure de diphénylèneiodonium, la 7-nitroindazole, la N(5)-(1-iminoéthyl)-L-ornithine, la N^{G},N^{G}-diméthyl-L-arginine, la N^{G},N^{G}-diméthyl-arginine, le 2-(4-carboxyphényl)-4,4,5,5-tetraméthyline-1-oxy-3-oxyde, l'aminoguanidine, la canavanine, l'ebselen et l'hormone stimulatrice des mélanocytes de type α.

Parmi les inhibiteurs de la NO-synthase, on utilise préférentiellement la N^{G}-monométhyl-L-arginine et l'hormone stimulatrice des mélanocytes de type α. Les inhibiteurs de la NO-synthase peuvent être utilisés seuls ou en mélange.

La bradykinine est un peptide d'origine plasmatique libéré à partir d'un précurseur kininogène par une protéase plasmatique du nom de Kallikreine (EC 3.4.21.24). Ce nanopeptide est un des médiateurs clés de l'inflammation et possède des propriétés mitogènes. Les récepteurs pour cette kinine se divisent en deux principaux sous types, B1 et B2. La bradykinine agit notamment, sur le récepteur B2 et provoque la stimulation de nombreux systèmes de production de seconds messagers dont l'hydrolyse des inositol-phosphates, du métabolisme de l'acide arachidonique, la phosphorylation des résidus tyrosine ainsi que la dépolarisation ou l'hyperpolarisation de la membrane cellulaire.

L'activation de certains récepteurs provoque l'activation de la phospholipase C et donc la production de l'inositol 1,4,5-triphosphate (IP3) et de diacylglycérol (DAG). L'IP3 est connu pour provoquer la libération de calcium à partir des sites de stockages intracellulaires dans les cellules dont le kératinocyte. Le calcium, décrit comme activateur et régulateur de nombreuses enzymes (protéases, phospholipases), joue un rôle important dans la régulation de la différenciation et de la prolifération du kératinocyte.
Par antagoniste de la bradykinine, on entend tout composé susceptible d'inhiber partiellement, voire totalement, l'effet biologique de la bradykinine.

Particulièrement, pour qu'une substance soit reconnue comme un antagoniste de la bradykinine elle doit induire une réponse pharmacologique cohérente incluant ou non sa fixation au récepteur de la bradykinine.

Ainsi, entre dans cette définition tout composé qui peut interférer avec les effets de la bradykinine par sa fixation au récepteur de celle-ci (B1 ou B2) et/ou tout composé qui indépendamment de la fixation au(x) récepteur(s) induit par un mécanisme quelconque un effet contraire à celui connu de la bradykinine (par exemple interférant avec la synthèse de la bradykinine).

Parmi les antagonistes de la bradykinine, on préfère utiliser des composés inhibant la synthèse et/ou accélérant le catabolisme de la bradykinine, des composés neutralisant la bradykinine, des composés bloquant les récepteurs de la bradykinine tels que ceux qui interférent avec les effets de la bradykinine par leur fixation au récepteur de celle-ci (B1 ou B2), des composés inhibant la synthèse des récepteurs de la bradykinine ou des composés intervenant en diminuant le signal transduit par la bradykinine. Ces composés peuvent être d'origine naturelle ou synthétique.

Parmi les antagonistes de la bradykinine, on peut citer plus particulièrement des peptides, synthétiques ou naturels, éventuellement modifiés, comme la D-Arg, [Hyp3, D-Phe7]-bradykinin (NPC567), la [Thi 5, 8, D-Phe7]-bradykinin, la D-Arg, [Hyp3, Thi5,8, D-Phe7]-bradykinin, la N-α-adamantaneacetyl-D-Arg, [Hyp3, Thi5,8, D-Phe7]-bradykinin, la des-Arg9, [Leu8]-bradykinin (tous vendus par la société Sigma) ou encore les composés cités dans les brevets WO 95/08566, WO 95/07294, EP 0623350, EP 0622361, WO 94/11021, EP 0596406, WO 94/06453, WO 94/09001, EP 0578521, EP 0564972, EP 0552106, WO 93/11789, US 5216165, US 5212182, WO 92/17201, EP 0496369, EP 0472220, EP 0455133, WO 91/09055, WO 91/02746, EP 0413277, EP 0370453, EP 0359310, WO 90/03980, WO 89/09231, WO 89/09230, WO 89/01780, EP 0334244, EP 0596406, WO 86/07263 ou la P-guanidobenzoyl, [Hyp3,Thi5,D-Tic7,Oic8]-bradyklnin (S 16118) (Feletou M & al., Pharmacol. Exp. Ther., June 1995, 273, 1078-84), la D-Arg, [Hyp3, Thi5, D-Tic7,Oic8]-bradykinine (HOE 140) (Feletou-M & al., Eur. J. Pharmacol. 1995, 274, 57-64), la D-Arg, [Hyp3, D-Hype (trans-propyl) 7, Oic 8]-bradykinin (NPC 17731) ( Herzig M.C.S. and Leeb-Lundberg L.M.F., J. Biol. Chem. 1995, 270, 20591-20598) ou ceux cités dans Bradykinin Antagonists : development and applications (Stewart J.M., Biopolymers, 1995, 37, 143-155), ou encore des molécules chimiques, synthétiques ou naturelles, comme par exemple celles décrites dans Salvino et coll. J. Med. Chem., 1993, 36, 2583-2584.

On peut également utiliser selon l'invention des acides nucléiques antisens ou des ribozymes ayant pour but de sélectivement inhiber la synthèse de la bradykinine. Ces acides nucléiques antisens sont connus de l'homme du métier. Ils peuvent agir de différentes manières sur l'ADN ou sur l'ARN messager codant pour la bradykinine, notamment par blocage de la fixation ou de la progression des ribosomes le long de l'ARN messager, par clivage de l'ARN messager par la RNase H, ou en empêchant le transport de l'ARN messager du noyau vers le cytoplasme ou encore en empêchant la maturation de l'ARN messager.

On peut encore utiliser selon l'invention des anticorps anti-bradykinine ou des récepteurs solubles de la bradykinine, des anticorps anti-récepteurs de la bradykinine ou des antagonistes des récepteurs de la bradykinine.

Préférentiellement, selon l'invention on utilise un composé qui interfère avec les effets de la bradykinine par sa fixation au récepteur de celle-ci (B1 ou B2), préférentiellement au récepteur B2.

Encore plus préférentiellement, on utilise selon l'invention un antagoniste de la bradykinine choisi parmi :
la D-Arg, [Hyp3, D-Phe7]-bradykinin (NPC567),
la [Thi 5, 8, D-Phe7]-bradykinin, la D-Arg, [Hyp3, Thi5,8, D-Phe7]-bradykinin,
la N-α-adamantaneacetyl-D-Arg, [Hyp3, Thi5,8, D-Phe7]-bradykinin,
la des-Arg9, [Leu8]-bradykinin,
la P-guanidobenzoyl, [Hyp3,Thi5,D-Tic7,Oic8]-bradyklnin (S 16118),
la D-Arg, [Hyp3, Thi5, D-Tic7,Oic8]-bradykinine (HOE 140),
la D-Arg, [Hyp3, D-Hype (trans-propyl) 7, Oic 8]-bradykinin (NPC 17731)
Le peptide modifié préférentiellement utilisé selon l'invention est la D-Arg, [Hyp3, Thi5, D-Tic7,Oic8]-bradykinine (HOE 140).

Les antagonistes de bradykinine peuvent être utilisés seuls ou en mélange.

On sait, par ailleurs, que la substance P libérée par les terminaisons sensitives épidermiques induit une cascade d'événements biochimiques dont les premières étapes se situent au niveau des mastocytes. La fixation de la substance P sur les récepteurs mastocytaires induit une libération de nombreux médiateurs pro-inflammatoires parmi lesquels l'histamine, les cytokines comme l'interleukine 1 (IL1), l'interleukine 6 (IL6), l'interleukine 8 (IL8) et le facteur de nécrose tumorale de type α (Tumor Necrosis Factor α (TNF-α)).

On entend par antagonistes d'histamine, de cytokines et/ou de TNF-α, toutes substances susceptibles d'inhiber la libération et/ou la synthèse et/ou la fixation réceptorielle respectivement d'histamine, de cytokines et/ou de TNF-α.

Les antagonistes inhibant la fixation réceptorielle de l'histamine sont des agents spécifiques du récepteur de type 1 de l'histamine (H1).

Pour qu'une substance soit reconnue comme un antagoniste réceptoriel d'histamine, de cytokines ou de TNF-α, elle doit répondre à l'une des caractéristiques suivantes :
- avoir une affinité pour les récepteurs spécifiques de ces composés ;
- avoir une activité pharmacologique antagoniste réceptoriel d'histamine, de cytokines ou de TNF-α, c'est-à-dire induire une réponse pharmacologique cohérente dans l'un des tests suivants :
   + pour les antagonistes réceptoriels d'histamine : une inhibition de la contraction des muscles lisses induite par l'administration d'histamine ;
   + pour les antagonistes réceptoriels de cytokines: inhibition de l'adhésion de macrophages induite par les cytokines sur les cellules endothéliales ou inhibition de la libération d'anions superoxydes induite par les cytokines sur les neutrophiles ;
   + pour les antagonistes réceptoriels de TNF-α : inhibition de l'adhésion de macrophages induite par TNF-α sur les cellules endothéliales ou inhibition de la libération d'anions superoxydes induite par TNF-α sur les neutrophiles ou inhibition de l'activité mitogène du TNF-α sur les fibroblastes du derme.

Pour qu'une substance soit reconnue comme un antagoniste de la libération et/ou de la synthèse d'histamine, de cytokines ou de TNF-α, elle doit répondre à l'une des caractéristiques suivantes :
- inhibition de la libération d'histamine par des mastocytes stimulés par le composé 48/80 ou stimulés par un ionophore calcique (A23 187)
- inhibition de la libération de cytokines ou de TNF-α par des monocytes (cellules U937) différenciés par un ester de phorbol (PMA).

Les antagonistes réceptoriels d'histamine H1 utilisables dans l'invention sont ceux classiquement utilisés dans les traitements des états allergiques et anaphylactiques ainsi que ceux pour lutter contre le mal des transports. Ces composés peuvent être par exemple des dérivés de la diéthylène diamine comme la Cinnarizine, la cyclizine ; des dérivés de l'aminopropane comme la dexchlorophéniramine, la triprolidine ; des dérivés de phénothiazine comme la prométhazine, l'alimémazine ; ainsi que les composés cités pages 116 à 118 du livre Joseph R. Prous, The Year's Drug News, Therapeutic Targets, édition 1994, Prous Science Publishers comme la cétirizine HCl, l'ébastine, la loratadine, la sétastine HCl.

Les inhibiteurs de libération de l'histamine sont notamment des composés hétérocycliques oxygénés ou soufrés tels que les dérivés du furanne, les dérivés du benzofuranne, les dérivés du thiophène et les dérivés du benzothiophène, comportant éventuellement des substituants azotés, tels que ceux décrits dans les documents US-A-4931459, US-A-4910317 et EP-A-299457, et plus spécialement les alcoxy- et/ou aryloxy- tétrazol-yl-benzofuranne-carboxamides ou les alcoxy-et/ou aryloxy- tétrazol-yl-benzothiophène-carboxamides. A titre d'exemple, on peut citer le 5-méthoxy-3-phénoxy-N-1H-tétrazol-5-yl-benzothiophène-2-carboxamide, le 5-méthoxy-3-(1-méthyléthoxy)-N-1H-tétrazol-5-yl-benzothiophène-2-carboxamide, le 6-méthoxy-3-(1-méthyléthoxy)-N-1H-tétrazol-5-yl-benzothiophène-2-carboxamide, le 5-méthoxy-3-(1-méthyléthyl)-N-1H-tétrazol-5-yl-benzothiophène-2-carboxamide, le 3-benzyloxy-5-méthoxy-N-1H-tétrazol-5-yl-benzothiophène-2-carboxamide, et le 5-méthoxy-3-phénoxy-N-1H-tétrazol-5-yl-benzothiophène-2-carboxamide.

Parmi les antagonistes de cytokines, on citera par exemple un antagoniste de la libération de l'interleukine 1 utilisable dans l'invention qui peut être l'auranofine ou le SKF-105809 ou encore un antagoniste de la synthèse d'interleukine 1 qui peut être la lactoférrine.

Les antagonistes réceptoriels de TNF-α et les inhibiteurs de la libération et/ou de la synthèse de TNF-α utilisables dans l'invention sont en particulier la lisophyline, l'A802715, la sulfasalazine.

Les antagonistes d'histamine, de cytokines et de TNF-α peuvent être synthétisés ou extraits de produits naturels (végétaux ou animaux).

Les antagonistes d'histamine, de cytokines et de TNF-α peuvent être utilisés selon l'invention, séparément ou associés, seuls ou sous forme de mélange.

La quantité de composé diminuant la synthèse, la libération et/ou l'activité d'au moins un médiateur de l'inflammation contenue dans la composition de l'invention est bien entendu fonction de l'effet recherché et peut donc varier dans une large mesure.

Pour donner un ordre de grandeur, la composition cosmétique de l'invention peut contenir un composé diminuant la synthèse, la libération et/ou l'activité d'au moins un médiateur de l'inflammation en une quantité représentant de 0,001% à 5% du poids total de la composition et préférentiellement en une quantité représentant de 0,01% à 2% du poids total de la composition.

Pour donner un ordre de grandeur, la composition pharmaceutique de l'invention peut contenir un composé diminuant la synthèse, la libération et/ou l'activité d'au moins un médiateur de l'inflammation en une quantité représentant de 0,001% à 10% du poids total de la composition et préférentiellement en une quantité représentant de 0,01% à 5% du poids total de la composition.

Quelque soit la forme de la composition selon l'invention, la quantité d'extrait d'au moins une Iridacée contenue dans la composition est bien entendu fonction de l'effet recherché et peut donc varier dans une large mesure.

Pour donner un ordre de grandeur, si la composition est une composition cosmétique, elle peut contenir un extrait d'au moins une Iridacée en une quantité représentant de 0,001% à 20% du poids total de la composition et préférentiellement en une quantité représentant de 0,1% à 10% du poids total de la composition.

Pour donner un ordre de grandeur, si la composition est une composition pharmaceutique, elle peut contenir un extrait d'au moins une Iridacée en une quantité représentant de 0,1% à 30% du poids total de la composition et préférentiellement en une quantité représentant de 0,5% à 20% du poids total de la composition.

La composition selon l'invention peut être ingérée, injectée ou appliquée sur la peau (sur toute zone cutanée du corps), les cheveux, les ongles ou les muqueuses (buccale, jugale, gingivale, génitale, conjonctive). Selon le mode d'administration, la composition selon l'invention peut se présenter sous toutes les formes galéniques normalement utilisées.

Pour une application topique sur la peau, la composition peut avoir la forme notamment de solution aqueuse ou huileuse ou de dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydre, ou encore de microcapsules ou microparticules, ou de dispersions vésiculaires de type ionique et/ou non ionique. Ces compositions sont préparées selon les méthodes usuelles.

Elles peuvent être également utilisées pour les cheveux sous forme de solutions aqueuses, alcooliques ou hydroalcooliques, ou sous forme de crèmes, de gels, d'émulsions, de mousses ou encore sous forme de compositions pour aérosol comprenant également un agent propulseur sous pression.

Pour l'injection, la composition peut se présenter sous forme de lotion aqueuse, huileuse ou sous forme de sérum. Pour les yeux, elle peut se présenter sous forme de gouttes et pour l'ingestion, elle peut se présenter sous forme de capsules, de granulés de sirops ou de comprimés.

Les quantités des différents constituants des compositions selon l'invention sont celles classiquement utilisées dans les domaines considérés.

Ces compositions constituent notamment des crèmes de nettoyage, de protection, de traitement ou de soin pour le visage, pour les mains, pour les pieds, pour les grands plis anatomiques ou pour le corps, (par exemple crèmes de jour, crèmes de nuit, crèmes démaquillantes, crèmes de fond de teint, crèmes anti-solaires), des fonds de teint fluides, des laits de démaquillage, des laits corporels de protection ou de soin, des laits anti-solaires, des lotions, gels ou mousses pour le soin de la peau, comme des lotions de nettoyage, des lotions anti-solaires, des lotions de bronzage artificiel, des compositions pour le bain, des compositions désodorisantes comprenant un agent bactéricide, des gels ou lotions après-rasage, des crèmes épilatoires, des compositions contre les piqûres d'insectes, des compositions anti-douleur, des compositions pour traiter certaines maladies de la peau comme l'eczéma, la rosacée, le psoriasis, les lichens, les prurits sévères.

Les compositions selon l'invention peuvent également consister en des préparations solides constituant des savons ou des pains de nettoyage.

Les compositions peuvent aussi être conditionnées sous forme de composition pour aérosol comprenant également un agent propulseur sous pression.

La composition selon l'invention peut aussi être une composition pour soins capillaires, et notamment un shampooing, une lotion de mise en plis, une lotion traitante, une crème ou un gel coiffant, une composition de teintures (notamment teintures d'oxydation) éventuellement sous forme de shampooings colorants, des lotions restructurantes pour les cheveux, une composition de permanente (notamment une composition pour le premier temps d'une permanente), une lotion ou un gel antichute, un shampooing antiparasitaire, etc.

La composition peut aussi être à usage bucco-dentaire, par exemple une pâte dentifrice. Dans ce cas, la composition peut contenir des adjuvants et additifs usuels pour les compositions à usage buccal et notamment des agents tensioactifs, des agents épaississants, des agents humectants, des agents de polissage tels que la silice, divers ingrédients actifs comme les fluorures, en particulier le fluorure de sodium, et éventuellement des agents édulcorants comme le saccharinate de sodium.

Lorsque la composition est une émulsion, la proportion de la phase grasse peut aller de 5 % à 80 % en poids, et de préférence de 5 % à 50 % en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique.

L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition est une solution ou un gel huileux, la phase grasse peut représenter plus de 90 % du poids total de la composition.

De façon connue, la composition cosmétique peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine cosmétique, et par exemple de 0,01 % à 10 % du poids total de la composition. Ces adjuvants, selon leur nature, peuvent être introduits dans la phase grasse, dans la phase aqueuse et/ou dans les sphérules lipidiques.

Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique).

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{R} 63 par la société Gattefosse.

Comme solvants utilisables dans l'invention, on peut citer les alcools inférieurs, notamment l'éthanol et l'isopropanol, le propylène glycol.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, polyéthylène.

La composition peut contenir d'autres actifs hydrophiles comme les protéines ou les hydrolysats de protéine, les acides aminés, les polyols, l'urée, l'allantoïne, les sucres et les dérivés de sucre, les vitamines hydrosolubles, les extraits végétaux et les hydroxyacides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

Selon l'invention la composition peut associer au moins un extrait d'au moins une Iridacée à d'autres agents actifs destinés notamment à la prévention et/ou au traitement des affections cutanées. Parmi ces agents actifs, on peut citer à titre d'exemple :
- les agents modulant la différenciation et/ou la prolifération et/ou la pigmentation cutanée tels que l'acide rétinoïque et ses isomères, le rétinol et ses esters, la vitamine D et ses dérivés, les oestrogènes tels que l'oestradiol, l'acide kojique ou l'hydroquinone ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents antiviraux tels que l'acyclovir ;
- les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique ;
- les agents anesthésiques tels que le chlorhydrate de lidocaïne et ses dérivés ;
- les agents antiprurigineux comme la thénaldine, la triméprazine ou la cyproheptadine ;
- les agents kératolytiques tels que les acides alpha- et bêta-hydroxycarboxyliques ou bêta-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'alpha-tocophérol ou ses esters, les superoxyde dismutases, certains chélatants de métaux ou l'acide ascorbique et ses esters ;
- les antiséborrhéiques tels que la progestérone ;
- les antipelliculaires comme l'octopirox ou la pyrithione de zinc ;
- les antiacnéiques comme l'acide rétinoïque ou le peroxyde de benzoyle.

Ainsi, selon un mode particulier, l'invention concerne une composition contenant au moins un extrait d'au moins une Iridacée et au moins un agent choisi parmi les agents antibactériens, antiparasitaires, antifongiques, antiviraux, anti-inflammatoires, antiprurigineux, anesthésiques, kératolytiques, anti-radicaux libres, anti-séborrhéiques, antipelliculaires, antiacnéiques et/ou les agents modulant la différenciation et/ou la prolifération et/ou la pigmentation cutanée.

La présente invention a en outre pour objet un procédé de traitement cosmétique non thérapeutique en vue de diminuer l'effet irritant d'une composition cosmétique, caractérisé par le fait que l'on applique sur la peau, sur les cheveux, et/ou sur les muqueuses, une composition cosmétique telle que décrite ci-dessus.

Le procédé de traitement cosmétique de l'invention peut être mis en oeuvre notamment en appliquant les compositions hygiéniques ou cosmétiques telles que définies ci-dessus, selon la technique d'utilisation habituelle de ces compositions. Par exemple : application de crèmes, de gels, de sérums, de lotions, de laits de démaquillage ou de compositions anti-solaires sur la peau ou sur les cheveux secs, application d'une lotion pour cheveux sur cheveux mouillés, de shampooings, ou encore application de dentifrice sur les gencives.

Les exemples et compositions suivants illustrent l'invention sans la limiter aucunement. Dans les compositions les proportions indiquées sont des pourcentages en poids.

### Exemple 1 : Préparation d'un extrait d'Iris pallida :

Des cellules indifférenciées d'*Iris pallida* obtenu par cultures *in vitro* en conditions axéniques sont récupérées après culture en Erlenmeyer ou en fermenteur par filtration sur tamis de 50µm. A 55 g de matière fraîche ainsi obtenue, on ajoute 27,5 ml d'eau déminéralisée. L'ensemble est broyé (Potter, Ultra Turrax...) au Turrax à 24000 T/min durant 1 minute à 4°C (bain de glace). Le broyat est centrifugé 15 à 10000 G à 4°C. Le surnageant est filtré à 0,22 µm (filtration stérilisante).
L'extrait ainsi préparé est conservé à 4°C. Il renferme environ 15 g de matière sèche par litre.
Si le matériel végétal est de la plante entière, on ramène la matière fraîche à traiter en fonction du poids sec pour se mettre dans les mêmes conditions d'extraction que pour l'*in vitro*. Les différentes parties de la plante sont prélevées en fonction du poids relatif de chaque partie de celle-ci. Le traitement à froid permet de geler les activités enzymatiques, la filtration stérilisante évite la dégradation des actifs par les micro-organismes de l'environnement. Enfin, le véhicule eau est compatible avec les récepteurs *ex vivo* et facilite les formulations cosmétiques ou pharmaceutiques.

### Exemple 2 : Mesure de l'affinité réceptorielle de l'extrait de cellules indifférenciées d'Iris pallida (exemple 1) pour le récepteur NK1 humain (récepteur à la substance P humain) :

### A) : Affinité réceptorielle :

La mesure de l'affinité réceptorielle de l'extrait de cellules indifférenciées d*'Iris pallida* pour le récepteur NK1 humain a été effectuée selon la méthode décrite dans l'article : Heuillet, E. et coll., J. Neurochem. ,60, 1993, 868-876.

L'extrait est testé aux concentrations de 1 %, 5 % et 10 %.

Lors de chaque expérience, la molécule de référence du récepteur étudié ([Sar⁹, Met (O₂)¹¹]-SP, analogue de la substance P décrit par Heuillet, E. (Heuillet, E. et al, J. Neurochem. ,60, 1993, 868-876)) est parallèlement testée à 8 concentrations (n = 2) pour l'obtention d'une courbe standard permettant de valider l'expérimentation.

On obtient ainsi :
26,8 % de fixation pour l'extrait de l'exemple 1 à 1 %
43,5% de fixation pour l'extrait de l'exemple 1 à 5 %
89,3% de fixation pour l'extrait de l'exemple 1 à 10 %

Les résultats de cette expérience mettent en évidence une affinité de l'extrait de cellules indifférenciées d*'Iris pallida* pour le récepteur à la substance P humain dès la concentration de 1 %.

La courbe d'affinité tracée d'après les résultats obtenus montre 50 % de déplacement du ligand naturel (IC50) par l'extrait d*'Iris pallida* à la concentration de 2 %.

### B) : Test fonctionnel in vitro :

Un test fonctionnel *in vitro* réalisé sur le récepteur NK1 humain (récepteur à la substance P humain) présents sur les muscles lisses d'intestin isolé (iléon) est réalisé pour mettre en évidence le caractère antagoniste de substance P de l'extrait de cellules indifférenciées d*'Iris pallida.*

Les expériences *in vitro* sont réalisées selon la méthode décrite par Dion et coll. (Life Sciences, 41, 1987, 2269-2278) et Patacchini et coll. (Eur. J. Pharmacol., 215, 1992, 93-98).

Après installation dans des cuves expérimentales, les tissus (muscles lisses) sont soumis à une tension initiale de 1 g. Une période d'équilibration d'au moins 60 minutes, au cours de laquelle la solution physiologique est plusieurs fois renouvelée et la tension initiale réajustée, est ensuite respectée avant l'addition de l'extrait.
Les expériences sont conduites en présence continue d'atropine (3.10⁻⁶ M), de pyrilamine (3.10⁻⁶ M) et d'indométhacine (10⁻⁶ M) pour s'affranchir des effets indirects de médiateurs mis en jeu lors de la stimulation d'autres types de récepteurs présents sur ce tissu.

Chaque préparation est initialement stimulée par un agoniste de substance P : [Sar⁹, Met (O₂)¹¹]-SP, à la concentration de 10⁻⁸ M pour l'obtention d'une réponse contractile "contrôle", puis la solution physiologique est entièrement renouvelée.

Cette opération est ensuite répétée toutes les 40 minutes en présence de concentrations croissantes de l'extrait d*'Iris pallida,* chacune d'elles étant ajoutée 30 minutes avant la [Sar⁹, Met (O₂)¹¹]-SP.

Une inhibition de 50 % de l'activité de la [Sar⁹, Met (O₂)¹¹]-SP est obtenue à la concentration de 7 % en extrait.

### C) : Test fonctionnel in vivo :

Un test fonctionnel *in vivo* est réalisé sur un modèle d'inflammation neurogène pour mettre en évidence le caractère antagoniste de substance P de l'extrait d*'Iris pallida* (exemple 1).

Les expériences *in vivo* sont réalisées selon la méthode décrite par Xu X.J. et collaborateurs (Neurosciences, 1991, 42, 731-737).

Le test consiste à provoquer une inflammation par la stimulation antidromique du nerf saphène chez l'animal anesthésié. Ce nerf innerve les territoires cutanés des pattes postérieures.

La stimulation provoque la libération à partir des terminaisons nerveuses de substance P, responsable en partie de l'inflammation neurogène.

L'inflammation neurogène est quantifiée par la mesure de la perméabilité tissulaire au bleu Evans, un marqueur de l'extravasation tissulaire de l'albumine plasmatique qui a lieu au cours de l'inflammation.
Ce modèle de référence est utilisé pour la recherche *in vivo* d'antagonistes de la substance P.

L'extrait d'*Iris pallida* sous sa forme aqueuse administré dilué au 1/10 ème provoque une diminution statistiquement significative de 38 % de l'inflammation neurogène.

### Conclusions :

L'extrait de cellules indifférenciées d'*Iris pallida* présente une affinité pour le récepteur de la substance P et exerce une activité spécifique antagoniste de la substance P.

### Exemple 3 : Mesure de l'affinité réceptorielle de l'extrait de cellules indifférenciées d'Iris pallida (exemple 1) pour le récepteur au CGRP.

### A) : Affinité réceptorielle :

La mesure de l'affinité réceptorielle de l'extrait de cellules indifférenciées d'*Iris pallida* de l'exemple 1 pour le récepteur au CGRP a été effectuée selon la méthode décrite dans l'article : Mimeault, M. et coll., J. Med. Chem., 35, 1992, 2163-2168.

L'extrait est testé aux concentrations de 0,5%, 1% et 5 %.

Lors de chaque expérience, la molécule de référence du récepteur étudié (le composé hCGRPα, analogue du CGRP décrit par Mimeault, M. (Mimeault, M. et coll., J. Med. Chem., 35, 1992, 2163-2168)) est parallèlement testée à 8 concentrations (n = 2) pour l'obtention d'une courbe standard permettant de valider l'expérimentation.

On obtient ainsi :
17 % de fixation pour l'extrait de l'exemple 1 à 0,5%
36 % de fixation pour l'extrait de l'exemple 1 à 1%
100 % de fixation pour l'extrait de l'exemple 1 à 5 %

Les résultats de cette expérience mettent en évidence une affinité de l'extrait de cellules indifférenciées d'*Iris pallida* pour le récepteur du CGRP humain dès la concentration de 0,5 %.

La courbe d'affinité tracée d'après les résultats obtenus montre 50 % de déplacement du ligand naturel (IC50) par l'extrait d'*Iris pallida* à la concentration de 2,5 %.

### B) : Test fonctionnel in vitro :

Un test fonctionnel *in vitro* réalisé sur les récepteurs au CGRP présents sur les muscles lisses de vas deferens est réalisé pour mettre en évidence le caractère antagoniste du CGRP de l'extrait d'*Iris pallida.*

Les expériences *ex vivo* sont réalisées selon la méthode décrite par Longmore et coll. (Eur. J. Pharmacol., 265, 1994, 53-59).

Après installation dans des cuves expérimentales, les tissus (muscles lisses) sont soumis à une tension initiale de 1 g. Une période d'équilibration d'au moins 60 minutes, au cours de laquelle la solution physiologique est plusieurs fois renouvelée et la tension initiale réajustée, est ensuite respectée.

Suite à cette période, une stimulation électrique est appliquée sur le muscle lisse de vas deferens, l'addition de l'extrait est effectué après stabilisation de la réponse contractile induite par cette stimulation, soit environ 30 minutes.

Les expériences sont conduites en présence continue de thiorphan (10⁻⁵ M) pour inhiber la dégradation du CGRP ou de ses analogues par les endopeptidases présentés sur ce tissu.

Chaque préparation est exposée au hCGRPα (analogue du CGRP) à la concentration de 10⁻⁸ M pour l'obtention d'une réponse inhibitrice contrôle.

Après stabilisation de cette réponse, des concentrations croissantes de l'extrait d'*Iris pallida* ou de hCGRPα, sont testés de façon cumulative, une récupération des contractions par ces composés indiquant un effet antagoniste sur les récepteurs CGRP.

L'extrait d'*Iris pallida* est testé aux 3 concentrations suivantes : 0,5 %, 1 % et 5 % de sa concentration initiale.

Les résultats obtenus sont comparés à ceux des molécules de référence. Le nombre de préparations utilisées dans chaque cas est de 2 (n =2).

Après inhibition préalable des contractions par le hCGRPα, l'extrait d'*Iris pallida* permet une récupération de ces dernières, effet qualitativement identique à celui exercé par le hCGRPα.

Une inhibition de 50 % de l'activité du hCGRPα, est obtenue à la concentration de 0,8% en extrait.

### Conclusions :

L'extrait de cellules indifférenciées d'*Iris pallida* présente une affinité pour le récepteur du CGRP et exerce une activité spécifique antagoniste du CGRP.

### Exemple 4 : Exemples de formulations illustrant l'invention. Ces compositions ont été obtenues par simple mélange des différents composants.

| Composition 1 : Lotion démaquillante pour le visage | |
|---|---|
| Extrait de l'exemple 1 | 5,00 |
| Antioxydant | 0,05 |
| Isopropanol | 40,00 |
| Conservateur | 0,30 |
| Eau | qsp 100 % |

| Composition 2 : Gel pour le soin du visage | |
|---|---|
| Extrait de l'exemple 1 | 0,50 |
| Hydroxypropylcellulose (Klucel H® vendu par la société Hercules) | 1,00 |
| Antioxydant | 0,05 |
| Isopropanol | 40,00 |
| Conservateur | 0,30 |
| Eau | qsp 100 % |

| Composition 3 : Crème de soin du visage (émulsion huile dans eau) | |
|---|---|
| Extrait de l'exemple 1 | 2,00 |
| Stéarate de glycérol | 2,00 |
| Polysorbate 60 (Tween 60 vendu par la société ICI) | 1,00 |
| Acide stéarique | 1,40 |
| Triéthanolamine | 0,70 |
| Carbomer | 0,40 |
| Fraction liquide du beurre de karité | 12,00 |
| Perhydrosqualène | 12,00 |
| Antioxydant | 0,05 |
| Parfum | 0,50 |
| Conservateur | 0,30 |
| Eau | qsp 100 % |

| Composition 4 : Shampooing | |
|---|---|
| Extrait de l'exemple 1 | 0,50 |
| Hydroxypropylcellulose (Klucel H® vendu par la société Hercules) | 1,00 |
| Parfum | 0,50 |
| Conservateur | 0,30 |
| Eau | qsp 100 % |

| Composition 5 : Gel anti-douleur | |
|---|---|
| Extrait de l'exemple 1 | 5,00 |
| Hydroxypropylcellulose (Klucel H® vendu par la société Hercules) | 1,00 |
| Antioxydant | 0,05 |
| Chlorhydrate de lidocaïne | 2,00 |
| Isopropanol | 40,00 |
| Conservateur | 0,30 |
| Eau | qsp 100 % |

| Composition 6 : Crème de soin antirides pour le visage (émulsion huile/eau) | |
|---|---|
| Extrait de l'exemple 1 | 0,15 |
| Stéarate de glycérol | 2,00 |
| Polysorbate 60 (Tween 60 vendu par la société ICI) | 1,00 |
| Acide stéarique | 1,40 |
| Acide n-octanoyl-5-salicylique | 0,50 |
| Triéthanolamine | 0,70 |
| Carbomer | 0,40 |
| Fraction liquide du beurre de karité | 12,00 |
| Perhydrosqualène | 12,00 |
| Antioxydant | 0,05 |
| Parfum | 0,5 |
| Conservateur | 0,300 |
| Eau | qsp 100 % |

| Composition 7 : Crème de soin de l'érythème solaire (émulsion huile-dans-eau) | |
|---|---|
| Extrait de l'exemple 1 | 2,50 |
| Stéarate de glycérol | 2,00 |
| Polysorbate 60 (Tween 60 vendu par la société ICI) | 1,00 |
| Acide stéarique | 1,40 |
| Acide glycyrrhétinique | 2,00 |
| Triéthanolamine | 0,70 |
| Carbomer | 0,40 |
| Fraction liquide du beurre de karité | 12,00 |
| Huile de tournesol | 10,00 |
| Antioxydant | 0,05 |
| Parfum | 0,50 |
| Conservateur | 0,30 |
| Eau | qsp 100 % |

| Composition 8 : Gel pour le traitement de l'acné | |
|---|---|
| Extrait de l'exemple 1 | 5,00 |
| Acide tout trans rétinoïque | 0,05 |
| Hydroxypropylcellulose (Klucel H®) | 1,00 |
| Antioxydant | 0,05 |
| Isopropanol | 40,00 |
| Conservateur | 0,30 |
| Eau | qsp 100 % |

| Composition 9 : Lotion pour éliminer les cicatrices dues à l'acné | |
|---|---|
| Extrait de l'exemple 1 | 0,50 |
| Acide glycolique | 50,00 |
| Hydroxypropylcellulose (Klucel H®) | 0,05 |
| NaOH | qsp pH = 2,8 |
| Ethanol | qsp 100 % |
| Conservateur | 0,30 |

| Composition 10 : Lotion démaquillante pour le visage | |
|---|---|
| Extrait de l'exemple 1 | 5,00 |
| Chlorure de strontium | 5,00 |
| Antioxydant | 0,05 |
| Isopropanol | 40,00 |
| Conservateur | 0,30 |
| Eau | qsp 100 % |

| Composition 11 : Gel pour le soin du visage | |
|---|---|
| Extrait de l'exemple 1 | 5,00 |
| Eau thermale de Vichy | 10,00 |
| Hydroxypropylcellulose (Klucel H® vendu par la société Hercules) | 1,00 |
| Antioxydant | 0,05 |
| Isopropanol | 40,00 |
| Conservateur | 0,30 |
| Eau | qsp 100 % |

| Composition 12 : Crème de soin du visage (émulsion huile dans eau) | |
|---|---|
| Extrait de l'exemple 1 | 5,00 |
| Auranofine | 0,10 |
| Stéarate de glycérol | 2,00 |
| Polysorbate 60 (Tween 60 vendu par la société ICI) | 1,00 |
| Acide stéarique | 1,40 |
| Triéthanolamine | 0,70 |
| Carbomer | 0,40 |
| Fraction liquide du beurre de karité | 12,00 |
| Perhydrosqualène | 12,00 |
| Antioxydant | 0,05 |
| Conservateur | 0,30 |
| Eau | qsp 100 % |

| Composition 13 : Shampooing | |
|---|---|
| Extrait de l'exemple 1 | 5,00 |
| Chlorure de strontium | 5,00 |
| Hydroxypropylcellulose (Klucel H® vendu par la société Hercules) | 1,00 |
| Parfum | 0,50 |
| Conservateur | 0,30 |
| Eau | qsp 100 % |

| Composition 14 : Lotion pour éliminer les cicatrices dues à l'acné | |
|---|---|
| Extrait de l'exemple 1 | 5,00 |
| HOE 140 | 0,05 |
| Acide glycolique | 50,00 |
| Hydroxypropylcellulose (Klucel H® vendu par la société Hercules) | 0,05 |
| NaOH | qsp pH = 2,8 |
| Ethanol | qsp 100 % |
| Conservateur | 0,30 |

| Composition 15 : Crème de soin de l'érythème solaire (émulsion huile-dans-eau) | |
|---|---|
| Extrait de l'exemple 1 | 5,00 |
| Eau thermale de Vichy | 10,00 |
| Stéarate de glycérol | 2,00 |
| Polysorbate 60 (Tween 60 vendu par la société ICI) | 1,00 |
| Acide stéarique | 1,40 |
| Acide glycyrrhétinique | 2,00 |
| Triéthanolamine | 0,70 |
| Carbomer | 0,40 |
| Fraction liquide du beurre de karité | 12,00 |
| Huile de tournesol | 10,00 |
| Antioxydant | 0,05 |
| Parfum | 0,50 |
| Conservateur | 0,30 |
| Eau | qsp 100 % |

| Composition 16 : Gel pour le traitement de l'acné | |
|---|---|
| Extrait de l'exemple 1 | 5,00 |
| CGRP 8-35 | 0,50 |
| Acide tout trans rétinoïque | 0,05 |
| Hydroxypropylcellulose (Klucel H® vendu par la société Hercules) | 1,00 |
| Antioxydant | 0,05 |
| Isopropanol | 40,00 |
| Conservateur | 0,30 |
| Eau | qsp 100 % |

| Composition 17 : Gel anti-douleur | |
|---|---|
| Extrait de l'exemple 1 | 5,00 |
| Spantide II | 0,05 |
| Hydroxypropylcellulose (Klucel H® vendu par la société Hercules) | 1,00 |
| Antioxydant | 0,05 |
| Chlorhydrate de lidocaïne | 2,00 |
| Isopropanol | 40,00 |
| Conservateur | 0,30 |
| Eau | qsp 100 % |

| Composition 18 : Crème de soin antirides pour le visage (émulsion huile/eau) | |
|---|---|
| Extrait de l'exemple 1 | 5,00 |
| Lactoférrine | 1,00 |
| Stéarate de glycérol | 2,00 |
| Polysorbate 60 (Tween 60 vendu par la société ICI) | 1,00 |
| Acide stéarique | 1,40 |
| Acide n-octanoyl-5-salicylique | 0,50 |
| Triéthanolamine | 0,70 |
| Carbomer | 0,40 |
| Fraction liquide du beurre de karité | 12,00 |
| Perhydrosqualène | 12,00 |
| Antioxydant | 0,05 |
| Parfum | 0,50 |
| Conservateur | 0,30 |
| Eau | qsp 100 % |

## Revendications

1. Procedé de traitement cosmétique non-thérapeutique des désordres associés à un excès de synthèse et/ou de libération du CGRP et/ou de substance P, caractérisé en ce que l'on utilise une composition cosmétique comprenant au moins un extrait de cellules d'au moins un végétal de la famille des Iridacées.

2. Procédé de traitement cosmétique non-thérapeutique selon la revendication 1, pour traiter les peaux sensibles.

3. Procédé de traitement cosmétique non-thérapeutique selon la revendication 1, pour prévenir et/ou lutter contre les irritations cutanées et/ou les érythèmes et/ou les sensations dysesthésiques et/ou les sensations d'échauffement de la peau et/ou des muqueuses.

4. Procédé de traitement cosmétique non-thérapeutique selon l'une quelconque des revendications précédentes, caractérisée en ce que l'extrait est préparé à partir de cellules provenant d'une Iridacée d'un genre choisi parmi Romulea, Crocus, Iris, Gladiolus, Sisyrinchium et Hermodactylus.

5. Procédé de traitement cosmétique non-thérapeutique selon la revendication précédente, caractérisée en ce que l'extrait est préparé à partir de cellules provenant d'une Iridacée du genre ris.

6. Procédé de traitement cosmétique non-thérapeutique selon la revendication précédente, caractérisée en ce que l'extrait est préparé à partir de cellules provenant d'Iris pallida.

7. Procédé de traitement cosmétique non-thérapeutique selon l'une quelconque des revendications précédentes, caractérisée en ce que l'extrait est préparé à partir de cellules provenant de matériel végétal issu de culture in vitro.

8. Procédé de traitement cosmétique non-thérapeutique selon l'une quelconque des revendications précédentes, caractérisée en ce que l'extrait est préparé à partir de cellules indifférenciées.

9. Utilisation d'au moins un extrait de cellules d'au moins un végétal de la famille des Iridacées pour la préparation d'une composition pharmaceutique pour le traitement de maladies susceptibles d'être traitées par des antagonistes du CGRP et/ou de substance P, à l'exception des troubles respiratoires.

10. Utilisation d'au moins un extrait de cellules d'au moins un végétal de la famille des Iridacées pour la préparation d'une composition pharmaceutique, destinée à traiter les désordres associés à un excès de synthèse et/ou de libération du CGRP et/ou de substance P, à l'exception des troubles respiratoires.

11. Utilisation d'au moins un extrait de cellules d'au moins une Iridacée pour la préparation d'une composition pharmaceutique pour traiter les désordres du système nerveux central, les syndromes allergiques, l'inflammation, la douleur, les désordres gastro-intestinaux, les désordres cutanés, les fibroses, les troubles de la maturation du collagène, les troubles cardio-vasculaires, les troubles vasospastiques, les désordres immunologiques et/ou les troubles du tractus urinaires.

12. Utilisation d'au moins un extrait de cellules d'au moins une Iridacée pour la préparation d'une composition pharmaceutique, pour prévenir et/ou lutter contre les dartres et/ou les prurits de la peau et/ou des muqueuses.

13. Utilisation selon l'une quelconque des revendications 9 à 12, caractérisée en ce que l'extrait est préparé à partir de cellules provenant d'une Iridacée d'un genre choisi parmi Romulea, Crocus, Iris, Gladiolus, Sisyrinchium et Hermodactylus.

14. Utilisation selon la revendication précédente, caractérisée en ce que l'extrait est préparé à partir de cellules provenant d'une Iridacée du genre Iris.

15. Utilisation selon la revendication précédente, caractérisée en ce que l'extrait est préparé à partir de cellules provenant d'Iris pallida.

16. Utilisation selon l'une quelconque des revendications précédentes, caractérisée en ce que l'extrait est préparé à partir de cellules provenant de matériel végétal issu de culture in vitro.

17. Utilisation selon l'une quelconque des revendications précédentes, caractérisée en ce que l'extrait est préparé à partir de cellules indifférenciées.

18. Composition cosmétique ou pharmaceutique, caractérisée en ce qu'elle comprend, dans un milieu cosmétiquement ou pharmaceutiquement acceptable, un extrait de cellules d'au moins un végétal de la famille des Iridacées et au moins un produit à effet irritant à l'exception de l'acide ascorbique.

19. Composition selon la revendication 18, caractérisée en ce que le produit à effet irritant est choisi parmi les tensioactifs ioniques ou non-ioniques, les conservateurs, les solvants organiques ou les actifs comme les α-hydroxy-acides, les β-hydroxy-acides, les α-céto-acides, les β-céto-acides, les rétinoïdes, les anthralines, les anthranoïdes, les peroxydes, le minoxidil, les sels de lithium, les antimétabolites, la vitamine D et ses dérivés, les teintures ou colorants capillaires, les solutions alcooliques parfumantes, les agents antitranspirants, les actifs dépilatoires, les actifs de permanentes, les actifs dépigmentants.

20. Composition cosmétique ou pharmaceutique, caractérisée en ce qu'elle comprend, dans un milieu cosmétiquement ou pharmaceutiquement acceptable, un extrait de cellules d'au moins un végétal de la famille des Iridacées et un composé diminuant la synthèse, la libération et/ou l'activité d'au moins un médiateur de l'inflammation, à l'exception de l'acide kojique.

21. Composition selon la revendication 20, caractérisée en ce que le composé diminuant la synthèse, la libération et/ou l'activité d'au moins un médiateur de l'inflammation est un composé diminuant la synthèse, la libération et/ou l'activité d'au moins un médiateur de l'inflammation cutanée.

22. Composition selon l'une quelconque des revendications 20 ou 21, caractérisée en ce que le composé diminuant la synthèse, la libération et/ou l'activité d'au moins un médiateur de l'inflammation est choisi parmi les antagonistes de substance P et/ou de CGRP, les inhibiteurs de NO-synthase, les antagonistes de bradykinine, les antagonistes de cytokines, les antagonistes d'histamine, les antagonistes du facteur de nécrose tumorale de type α (TNF α).

23. Composition selon la revendication 22, caractérisée en ce que les antagonistes sont des antagonistes réceptoriels.

24. Composition selon l'une quelconque des revendications 22 ou 23, caractérisée en ce que l'antagoniste de substance P est choisi parmi les substances assurant une diminution de l'extravasation du plasma au travers de la paroi vasculaire induite par la capsaïcine ou par une stimulation nerveuse antidromique et les substances provoquant une inhibition de la contraction des muscles lisses induites par l'administration de substance P.

25. Composition selon l'une quelconque des revendications 22 à 24, caractérisée en ce que l'antagoniste de substance P est choisi parmi les peptides, les composés comprenant au moins un hétérocycle, les composés azotés comprenant au moins un cycle benzénique, les sels de cations monovalents, divalents et trivalents, les eaux thermales et leurs mélanges.

26. Composition selon la revendication 25, caractérisée en ce que le peptide est le sendide ou le spantide II.

27. Composition selon la revendication 25, caractérisée en ce que le composé comprenant au moins un hétérocycle est un composé hétérocyclique azoté oxygéné ou soufré choisi parmi les dérivés de 2-tricyclyl-2-amino-éthane, les dérivés de spirolactame, les dérivés de quinuclidine, les dérivés azacycliques, les dérivés d'aminopyrrolidine, les dérivés de pipéridine, les aminoazahétérocycles, les dérivés d'isoindole, les dérivés du furanne, les dérivés du benzofuranne, les dérivés du thiophène et les dérivés du benzothiophène, et notamment les tétrazolylbenzofuranne-carboxamides ou les tétrazolyl-benzothiophène-carboxamides.

28. Composition selon la revendication 25, caractérisée en ce que le sel est choisi parmi les chlorures, acétates, carbonates, bicarbonates, salicylates, borates, nitrates, hydroxydes, sulfates, persulfates, des glycérophosphates, les sels d'α-hydroxyacides, les sels d'acides de fruits, les sels d'acides aminés et les sels d'acides gras de strontium, de magnésium, de lanthanides de numéro atomique allant de 57 à 71, de cobalt, de nickel, de manganèse, de baryum, d'yttrium, de cuivre, d'étain, de rubidium, de lithium et de zinc.

29. Composition selon la revendication 28, caractérisée en ce que le sel est choisi parmi les sels de strontium.

30. Composition selon l'une quelconque des revendications 28 ou 29, caractérisée en ce que le sel est du chlorure ou du nitrate de strontium.

31. Composition selon la revendication 25, caractérisée en ce que l'eau thermale est une eau provenant d'une source du bassin de Vichy.

32. Composition selon la revendication 31, caractérisée en ce que l'eau thermale provient des sources Célestins, Chomel, Grande-Grille, Hôpital, Lucas et Parc.

33. Composition selon l'une quelconque des revendications 31 ou 32, caractérisée en ce que l'eau thermale provient de la source Lucas.

34. Composition selon l'une quelconque des revendications 22 ou 23, caractérisée en ce que l'antagoniste de CGRP est choisi parmi les substances assurant une diminution de la vasodilatation induite par la capsaïcine ou par une stimulation électrique antidromique (appliquée sur un nerf afférent) et/ou une inhibition de la libération de CGRP par les fibres nerveuses sensitives et les substances provoquant une inhibition de la contraction du muscle lisse du vas deferens induite par le CGRP.

35. Composition selon la revendication 34, caractérisée en ce que l'antagoniste de CGRP est choisi parmi le CGRP 8-37 (séquence des acides aminés 8 à 37 de la partie N-terminale du CGRP) ou les anticorps anti-CGRP.

36. Composition selon la revendication 22, caractérisée en ce que l'inhibiteur de NO-synthase est choisi parmi les substances qui permettent in situ sur l'homme d'inhiber partiellement, voire totalement, la synthèse de monoxyde d'azote (NO).

37. Composition selon la revendication 36, caractérisée en ce que l'inhibiteur de NO-synthase est choisi parmi les composés inhibant la synthèse et/ou accélérant le catabolisme de la NO-synthase, les composés neutralisant la NO-synthase ou les composés intervenant en diminuant le signal transduit par la NO-synthase.

38. Composition selon l'une quelconque des revendications 36 ou 37, caractérisée en ce que l'inhibiteur de la NO-synthase est choisi parmi des peptides, synthétiques ou naturels, éventuellement modifiés, des molécules chimiques, synthétiques ou naturelles, des acides nucléiques antisens, des ribozymes, des anticorps anti-NO-synthase.

39. Composition selon l'une quelconque des revendications 36 à 38, caractérisée en ce que l'inhibiteur de la NO-synthase est choisi parmi la N^{G}-monométhyl-L-arginine (L-NMMA), la N^{G}-nitro-L-arginine, l'ester méthylé de la N^{G}-nitro-L-arginine, le chlorure de diphénylèneiodonium, la 7-nitroindazole, la N(5)-(1-iminoéthyl)-L-ornithine, la N^{G},N^{G}-diméthyl-L-arginine, la N^{G},N^{G}-diméthyl-arginine, le 2-(4-carboxyphényl)-4,4,5,5-tetraméthylimidazoline-1-oxy-3-oxyde, l'ebselen, l'aminoguanidine, la canavanine et l'hormone stimulatrice des mélanocytes de type α.

40. Composition selon l'une quelconque des revendications 36 à 38, caractérisèe en ce que l'inhibiteur de la NO-synthase est la N^{G}-monométhyl-L-arginine et l'hormone stimulatrice des mélanocytes de type α.

41. Composition selon l'une quelconque des revendications 22 ou 23, caractérisée en ce que l'antagoniste de la bradykinine est choisi parmi les composés inhibant la synthèse et/ou accélérant le catabolisme de la bradykinine, les composés neutralisant la bradykinine, les composés bloquant les récepteurs de la bradykinine tels que ceux qui interférent avec les effets de la bradykinine par leur fixation au récepteur de celle-ci (B1 ou B2), les composés inhibant la synthèse des récepteurs de la bradykinine ou les composés intervenant en diminuant le signal transduit par la bradykinine.

42. Composition selon la revendication 41, caractérisée en ce que l'antagoniste de la bradykinine est choisi parmi des peptides, synthétiques ou naturels, éventuellement modifiés, des molécules chimiques, synthétiques ou naturelles, des acides nucléiques antisens, des ribozymes, des anticorps anti-bradykinines, des récepteurs solubles de la bradykinine, des anticorps anti-récepteurs de la bradykinine ou des antagonistes des récepteurs de la bradykinine.

43. Composition selon l'une quelconque des revendications 41 ou 42, caractérisée en ce que l'antagoniste de la bradykinine est choisi parmi les composés qui interférent avec les effets de la bradykinine par leur fixation au récepteur de celle-ci (B1 ou B2) et préférentiellement au récepteur B2.

44. Composition selon l'une quelconque des revendications 41 à 43, caractérisée en ce que l'antagoniste de la bradykinine est choisi parmi la D-Arg, [Hyp3, D-Phe7]-bradykinin (NPC567), la [Thi 5, 8, D-Phe7]-bradykinin, la D-Arg, [Hyp3, Thi5,8, D-Phe7]-bradykinin, la N-α-adamantaneacetyl-D-Arg, [Hyp3, Thi5,8, D-Phe7]-bradykinin, la des-Arg9, [Leu8]-bradykinin, la P-guanidobenzoyl, [Hyp3,Thi5,D-Tic7,Oic8]-bradykinin (S 16118), la D-Arg, [Hyp3, Thi5, D-Tic7,Oic8]-bradykinin (HOE 140), la D-Arg, [Hyp3, D-Hype (trans-propyl) 7, Oic 8]-bradykinin (NPC 13731).

45. Composition selon l'une quelconque des revendications 41 à 44, caractérisée en ce que l'antagoniste de la bradykinine est la D-Arg, [Hyp3, Thi5, D-Tic7,Oic8]-bradykinine (HOE 140).

46. Composition selon l'une quelconque des revendications 22 ou 23, caractérisée en ce que l'antagoniste d'histamine, de cytokines ou de TNF-α est une substance choisie parmi les antagonistes réceptoriels d'histamine, de cytokines ou de TNF-a, ou parmi les antagonistes de la libération et/ou de la synthèse d'histamine, de cytokines ou de TNF-a

47. Composition selon la revendication 46, caractérisée en ce que la substance choisie parmi les antagonistes réceptoriels d'histamine, de cytokines ou de TNF-α est une substance ayant une affinité sélective pour les récepteurs spécifiques de ces composés ou assurant une inhibition de la contraction des muscles lisses induites par l'administration d'histamine ou assurant une inhibition de l'adhésion de macrophages induite par les cytokines sur les cellules endothéliales ou une inhibition de la libération d'anions superoxydes induite par les cytokines sur les neutrophiles ou une inhibition de l'adhésion de macrophages induite par TNF-α sur les cellules endothéliales ou une inhibition de la libération d'anions superoxydes induite par TNF-α sur les neutrophiles ou inhibition de l'activité mitogène du TNF-α sur les fibroblastes du derme.

48. Composition selon l'une quelconque des revendications 46 ou 47, caractérisée en ce que l'antagoniste réceptoriel d'histamine est choisi parmi les dérivés de la diéthylène diamine comme la Cinnarizine, la cyclizine ; les dérivés de l'aminopropane comme la dexchloro-phéniramine, la tripolidine ; les dérivés de phénothiazine comme la prométhazine, l'alimémazine ; la cétirizine HCl, l'ébastine, la loratadine, la sétastine HCl.

49. Composition selon la revendication 46, caractérisée en ce que l'antagoniste de la libération de l'histamine est choisi parmi des composés hétérocycliques oxygénés ou soufrés tels que les dérivés du furanne, les dérivés du benzofuranne, les dérivés du thiophène et les dérivés du benzothiophène, comportant éventuellement des substituants azotés, préférentiellement les alcoxy- et/ou aryloxy-tétrazol-yl-benzofuranne-carboxamides ou les alcoxy- et/ou aryloxy- tétrazol-yl-benzothiophène-carboxamides.

50. Composition selon l'une quelconque des revendications 46 à 49, caractérisée en ce que l'antagoniste de libération de l'histamine est choisi parmi le 5-méthoxy-3-phénoxy-N-1H-tétrazol-5-yl-benzothiophène-2-carboxamide, le 5-méthoxy-3-(1 - méthyléthoxy)-N-1H-tétrazol-5-yl-benzothiophène-2-carboxamide, le 6-méthoxy-3-( 1 - méthyléthoxy)-N-1H-tétrazol-5-yl-benzothiophène-2-carboxamide, le 5-méthoxy-3-(1 - méthyléthyl)-N-1H-tétrazol-5-yl-benzothiophène-2-carboxamide, le 3-benzyloxy-5-méthoxy-N-1H-tétrazol-5-yl-benzothiophène-2-carboxamide, le 5-méthoxy-3-phénoxy-N-1H-tétrazol-5-yl-benzothiophène-2-carboxamide.

51. Composition selon la revendication 46, caractérisée en ce que l'antagoniste de cytokines est choisi parmi les antagonistes de la libération de l'interleukine 1 comme l'auranofine ou le SKF-105809 ou les antagonistes de la synthèse d'interleukine 1 comme la lactoférrine.

52. Composition selon la revendication 46, caractérisée en ce que l'antagoniste réceptoriel de TNF-α et les inhibiteurs de la libération et/ou de la synthèse de TNF-α sont choisis parmi la lisophyline, l'A802715, la sulfasalazine.

53. Composition cosmétique selon l'une quelconque des revendications 18 à 52, caractérisée en ce que le composé diminuant la synthèse, la libération et/ou l'activité d'au moins un médiateur de l'inflammation est en une quantité représentant de 0,001 % à 5% du poids total de la composition et préférentiellement en une quantité représentant de 0,01% à 2% du poids total de la composition.

54. Composition pharmaceutique selon l'une quelconque des revendications 18 à 52, caractérisée en ce que le composé diminuant la synthèse, la libération et/ou l'activité d'au moins un médiateur de l'inflammation est en une quantité représentant de 0,001% à 10% du poids total de la composition et préférentiellement en une quantité représentant de 0,01% à 5% du poids total de la composition.

55. Composition pharmaceutique selon l'une quelconque des revendications 18 à 52 et 54, destinée à traiter les désordres du système nerveux central, les troubles respiratoires, les syndromes allergiques, l'inflammation, la douleur, les désordres gastro-intestinaux, les désordres cutanés, les fibroses, les troubles de la maturation du collagène les troubles cardio-vasculaires, les troubles vasospastiques, les désordres immunologiques et/ou les troubles du tractus urinaires.

56. Composition cosmétique selon l'une quelconque des revendications 18 à 53, destinée à prévenir et/ou lutter contre les irritations cutanées et/ou les érythèmes et/ou les sensations dysesthésiques et/ou les sensations d'échauffement de la peau et/ou les muqueuses.

57. Composition pharmaceutique selon l'une quelconque des revendications 18 à 52 ou 54 ou 55, destinée à prévenir et/ou lutter contre les dartres et/ou les prurits de la peau et/ou les muqueuses.

58. Composition selon l'une quelconque des revendications 20 à 57, caractérisée en ce qu'elle comprend en outre, dans un milieu cosmétiquement ou pharmaceutiquement acceptable, au moins un produit à effet irritant.

59. Composition selon l'une quelconque des revendications 18 à 58, caractérisée en ce que l'extrait de cellules d'au moins un végétal de la famille des Iridacées est tel que défini dans les revendications 1 à 10.

60. Composition cosmétique selon l'une quelconque des revendications 18 à 53 ou 56 ou 58 ou 59, caractérisée en ce que ledit extrait d'Iridacées est utilisé en une quantité représentant de 0,001% à 20% du poids total de la composition et préférentiellement en une quantité représentant de 0,1% à 10% du poids total de la composition.

61. Composition pharmaceutique selon l'une quelconque des revendications 18 à 52 ou 54 ou 55 ou 57 à 59, caractérisée en ce que ledit extrait d'Iridacées est utilisé en une quantité représentant de 0,1% à 30% du poids total de la composition et préférentiellement en une quantité représentant de 0,5% à 20% du poids total de la composition.

62. Procédé de traitement cosmétique non thérapeutique, caractérisé en ce que l'on applique sur la peau, sur les cheveux et/ou sur les muqueuses une composition cosmétique telle que définie dans l'une quelconque des revendications 18 à 53 ou 56 ou 58 à 60.

## Claims

1. Process for the non-therapeutic cosmetic treatment of disorders associated with an excessive synthesis and/or release of CGRP and/or substance P, characterized in that a cosmetic composition comprising at least one extract of cells of at least one plant of the Iridaceae family is used.

2. Process of non-therapeutic cosmetic treatment according to Claim 1, for treating sensitive skins.

3. Process of non-therapeutic cosmetic treatment according to Claim 1, for preventing and/or combating skin irritations and/or erythemas and/or dysaesthetic sensations and/or sensations of inflammation of the skin and/or of the mucous membranes.

4. Process of non-therapeutic cosmetic treatment according to any one of the preceding claims, characterized in that the extract is prepared from cells obtained from an Iridaceae of a genus chosen from Romulea, Crocus, Iris, Gladiolus, Sisyrinchium and Hermodactylus.

5. Process of non-therapeutic cosmetic treatment according to the preceding claim, characterized in that the extract is prepared from cells obtained from an Iridaceae of the genus Iris.

6. Process of non-therapeutic cosmetic treatment according to the preceding claim, characterized in that the extract is prepared from cells obtained from Iris pallida.

7. Process of non-therapeutic cosmetic treatment according to any one of the preceding claims, characterized in that the extract is prepared from cells obtained from plant material resulting from in vitro culture.

8. Process of non-therapeutic cosmetic treatment according to any one of the preceding claims, characterized in that the extract is prepared from undifferentiated cells.

9. Use of at least one extract of cells of at least one plant of the Iridaceae family for the preparation of a pharmaceutical composition for the treatment of diseases which can be treated with antagonists of CGRP and/or substance P, with the exception of respiratory disorders.

10. Use of at least one extract of cells of at least one plant of the Iridaceae family for the preparation of a pharmaceutical composition, intended to treat disorders associated with an excessive synthesis and/or release of CGRP and/or substance P with the exception of respiratory disorders.

11. Use of at least one extract of cells of at least one Iridaceae for the preparation of a pharmaceutical composition for treating disorders of the central nervous system, allergic syndromes, inflammation, pain, gastrointestinal disorders, skin disorders, fibrosis, collagen maturation disorders, cardiovascular disorders, vasospastic disorders, immunological disorders and/or disorders of the urinary tract.

12. Use of at least one extract of cells of at least one Iridaceae for the preparation of a pharmaceutical composition, to prevent and/or combat dartres and/or pruritus of the skin and/or of the mucous membranes.

13. Use according to any one of Claims 9 to 12, characterized in that the extract is prepared from cells obtained from an Iridaceae of a genus chosen from Romulea, Crocus, Iris, Gladiolus, Sisyrinchium and Hermodactylus.

14. Use according to the preceding claim, characterized in that the extract is prepared from cells obtained from an Iridaceae of the genus Iris.

15. Use according to the preceding claim, characterized in that the extract is prepared from cells obtained from Iris pallida.

16. Use according to any one of the preceding claims, characterized in that the extract is prepared from cells obtained from plant material resulting from in vitro culture.

17. Use according to any one of the preceding claims, characterized in that the extract is prepared from undifferentiated cells.

18. Cosmetic or pharmaceutical composition, characterized in that it comprises, in a cosmetically or pharmaceutically acceptable medium, an extract of cells of at least one plant of the Iridaceae family and at least one product with an irritant effect with the exception of ascorbic acid.

19. Composition according to Claim 18, characterized in that the product with an irritant effect is chosen from ionic or nonionic surfactants, preservatives, organic solvents or active agents such as α-hydroxy acids, β-hydroxy acids, α-keto acids, β-keto acids, retinoids, anthralins, anthranoids, peroxides, minoxidil, lithium salts, antimetabolites, vitamin D and its derivatives, hair colorants or dyes, perfuming alcoholic solutions, antiperspirant agents, depilatory active agents, active agents for permanent waving, depigmenting active agents.

20. Cosmetic or pharmaceutical composition, characterized in that it comprises, in a cosmetically or pharmaceutically acceptable medium, an extract of cells of at least one plant of the Iridaceae family and a compound reducing the synthesis, release and/or activity of at least one inflammation mediator with the exception of kojic acid.

21. Composition according to Claim 20, characterized in that the compound reducing the synthesis, release and/or activity of at least one inflammation mediator is a compound reducing the synthesis, release and/or activity of at least one skin inflammation mediator.

22. Composition according to either of Claims 20 and 21, characterized in that the compound reducing the synthesis, release and/or activity of at least one inflammation mediator is chosen from antagonists of substance P and/or CGRP, inhibitors of NO-synthase, antagonists of bradykinin, antagonists of cytokines, antagonists of histamine, antagonists of type α tumour necrosis factor (TNF α).

23. Composition according to Claim 22, characterized in that the antagonists are receptor antagonists.

24. Composition according to either of Claims 22 and 23, characterized in that the substance P antagonist is chosen from the substances which bring about a reduction in the extravasation of plasma across the vascular wall induced by capsaicin or by an antidromic nerve stimulation, and the substances which cause an inhibition of the contraction of the smooth muscles induced by the administration of substance P.

25. Composition according to any one of Claims 22 to 24, characterized in that the substance P antagonist is chosen from peptides, compounds comprising at least one heterocycle, nitrogen-containing compounds comprising at least one benzene ring, salts of monovalent, divalent and trivalent cations, thermal waters and mixtures thereof.

26. Composition according to Claim 25, characterized in that the peptide is Sendide or Spantide II.

27. Composition according to Claim 25, characterized in that the compound comprising at least one heterocycle is a nitrogen-, oxygen- or sulphur-containing heterocyclic compound chosen from 2-tricyclyl-2-amino-ethane derivatives, spirolactam derivatives, guinuclidine derivatives, azacyclic derivatives, aminopyrrolidine derivatives, piperidine derivatives, aminoazaheterocycles, isoindole derivatives, furan derivatives, benzofuran derivatives, thiophene derivatives, benzothiophene derivatives, and especially tetrazolyl-benzofuran-carboxamides or tetrazolyl-benzothiophene-carboxamides.

28. Composition according to Claim 25, characterized in that the salt is chosen from the chlorides, acetates, carbonates, bicarbonates, salicylates, borates, nitrates, hydroxides, sulphates, persulphates, glycerophosphates, salts of α-hydroxy acids, salts of fruit acids, salts of amino acids and salts of fatty acids, of strontium, magnesium, lanthanides of atomic number ranging from 57 to 71, cobalt, nickel, manganese, barium, yttrium, copper, tin, rubidium, lithium and zinc.

29. Composition according to Claim 28, characterized in that the salt is chosen from strontium salts.

30. Composition according to either of Claims 28 and 29, characterized in that the salt is strontium chloride or nitrate.

31. Composition according to Claim 25, characterized in that the thermal water is a water obtained from a spring from the Vichy basin.

32. Composition according to Claim 31, characterized in that the thermal water is obtained from the Celestins, Chomel, Grande-Grille, Hôpital, Lucas and Parc springs.

33. Composition according to either or Claims 31 and 32, characterized in that the thermal water is obtained from the Lucas spring.

34. Composition according to either of Claims 22 and 23, characterized in that the CGRP antagonist is chosen from the substances which bring about a reduction in the vasodilation induced by capsaicin or by an antidromic electric stimulus (applied to an afferent nerve) and/or an inhibition of the release of CGRP by the sensitive nerve fibres and the substances which cause an inhibition of the contraction of the smooth muscle of the vas deferens induced by CGRP.

35. Composition according to Claim 34, characterized in that the CGRP antagonist is chosen from CGRP 8-37 (sequence of amino acids 8 to 37 of the N-terminal part of CGRP) or the anti-CGRP antibodies.

36. Composition according to Claim 22, characterized in that the inhibitor of NO-synthase is chosen from the substances which make it possible in situ, in man, to inhibit partially or completely the synthesis of nitrogen monoxide (NO).

37. Composition according to Claim 36, characterized in that the inhibitor of NO-synthase is chosen from the compounds inhibiting the synthesis and/or accelerating the catabolism of NO-synthase, the compounds neutralizing NO-synthase or the compounds playing a role in reducing the signal transduced by NO-synthase.

38. Composition according to either of Claims 36 and 37, characterized in that the inhibitor of NO-synthase is chosen from optionally modified synthetic or naturally occurring peptides, synthetic or naturally occurring chemical molecules, antisense nucleic acids, ribozymes, anti-NO-synthase antibodies.

39. Composition according to any one of Claims 36 to 38, characterized in that the inhibitor of NO-synthase is chosen from N^{G}-monomethyl-L-arginine (L-NMMA), N^{G}-nitro-L-arginine, the methylated ester of N^{G}-nitro-L-arginine, diphenyleneiodonium chloride, 7-nitroindazole, N(5)-(1-iminoethyl)-L-ornithine, N^{G},N^{G}-dimethyl-L-arginine, N^{G},N^{G}-dimethylarginine, [2-(4-carboxyphenyl)-4,4,5,5-tetra-methylimidazoline-1-oxy-3-oxide, ebselen, aminoguanidine, canavanine, and type α melanocyte-stimulating hormone.

40. Composition according to any one of Claims 36 to 38, characterized in that the inhibitor of NO-synthase is N^{G}-monomethyl-L-arginine and the type α melanocyte-stimulating hormone.

41. Composition according to either of Claims 22 and 23, characterized in that the bradykinin antagonist is chosen from the compounds inhibiting the synthesis and/or accelerating the catabolism of bradykinin, compounds neutralizing bradykinin, compounds blocking the bradykinin receptors such as those which interfere with the effects of bradykinin by their attachment to the receptor for the latter (B1 or B2), compounds inhibiting the synthesis of the receptors for bradykinin or compounds which play a role in decreasing the signal transduced by bradykinin.

42. Composition according to Claim 41, characterized in that the bradykinin antagonist is chosen from optionally modified synthetic or naturally occurring peptides, synthetic or naturally occurring chemical molecules, antisense nucleic acids, ribozymes, anti-bradykinin antibodies, or soluble receptors for bradykinin, anti-bradykinin receptor antibodies or antagonists of bradykinin receptors.

43. Composition according to either of Claims 41 and 42, characterized in that the bradykinin antagonist is chosen from the compounds which interfere with the effects of bradykinin by their attachment to the receptor for the latter (B1 or B2) and preferably to the B2 receptor.

44. Composition according to any one of Claims 41 to 43, characterized in that the bradykinin antagonist is chosen from D-Arg-[Hyp3, D-Phe7]-bradykinin (NPC567), [Thi 5, 8, D-Phe7]-bradykinin, D-Arg, [Hyp3, Thi5, 8, D-Phe7]-bradykinin, N-α-adamantaneacetyl-D-Arg-[Hyp3, Thi5,8, D-Phe7]-bradykinin, des-Arg9, [Leu8]-bradykinin, P-guanidobenzoyl-[Hyp3,Thi5,D-Tic7,Oic8]-bradykinin (S 16118), D-Arg-[Hyp3, Thi5, D-Tic7, Oic8]-bradykinin (HOE 140), D-Arg-[Hyp3, D-Hype (trans-propyl)7 Oic8]-bradykinin (NPC 13731).

45. Composition according to any one of Claims 41 to 44, characterized in that the bradykinin antagonist is D-Arg-(Hyp3, Thi5, D-Tic7,Oic8]-bradykinin (HOE 140).

46. Composition according to either of Claims 22 and 23, characterized in that the antagonist of histamine, cytokines or TNF-α is a substance chosen from the receptor antagonists of histamine, cytokines or TNF-α, or from the antagonists of the release and/or synthesis of histamine, cytokines or TNF-α.

47. Composition according to Claim 46, characterized in that the substance chosen from the receptor antagonists of histamine, cytokines or TNF-α is a substance having a selective affinity for the specific receptors for these compounds or bringing about an inhibition of the contraction of the smooth muscles induced by the administration of histamine or bringing about an inhibition of the adhesion of macrophages which is induced by the cytokines on the endothelial cells or an inhibition of the release of superoxide anions which is induced by the cytokines on the neutrophils or an inhibition of the adhesion of macrophages which is induced by TNF-α on the endothelial cells or an inhibition of the release of superoxide anions which is induced by TNF-α on the neutrophils or inhibition of the mitogenic activity of TNF-α on the fibroblasts of the dermis.

48. Composition according to either of Claims 46 or 47, characterized in that the receptor antagonist of histamine is chosen from diethylenediamine derivatives such as cinnarizine or cyclizine; aminopropane derivatives such as dexchloropheniramine, tripolidine; phenothiazine derivatives such as promethazine, alimemazine; cetirizine HCl, ebastine, loratadine, setastine HCl.

49. Composition according to Claim 46, characterized in that the antagonist of the release of histamine is chosen from oxygen-containing or sulphur- containing heterocyclic compounds such as furan derivatives, benzofuran derivatives, thiophene derivatives and benzothiophene derivatives, optionally comprising nitrogen-containing substituents, preferably alkoxy- and/or aryloxytetrazol-yl-benzofuran-carboxamides or alkoxy- and/or aryloxy-tetrazol-yl-benzothiophene-carboxamides.

50. Composition according to any one of Claims 46 to 49, characterized in that the antagonist of the release of histamine is chosen from 5-methoxy-3-phenoxy-N-1H-tetrazol-5-yl-benzothiophene-2-carboxamide, 5-methoxy-3-(1-methylethoxy)-N-1H-tetrazol-5-yl-benzothiophene-2-carboxamide, 6-methoxy-3-(1-methylethoxy)-N-1H-tetrazol-5-yl-benzothiophene-2-carboxamide, 5-methoxy-3-(1-methyl-ethyl)-N-1H-tetrazol-5-yl-benzothiophene-2-carboxamide, 3-benzyloxy-5-methoxy-N-1H-tetrazol-5-yl-benzothiophene-2-carboxamide, and 5-methoxy-3-phenoxy-N-1H-tetrazol-5-yl-benzothiophene-2-carboxamide.

51. Composition according to Claim 46, characterized in that the antagonist of cytokines is chosen from the antagonists of the release of interleukin-1 such as auranofin or SKF-105809 or the antagonists of the synthesis of interleukin-1 such as lactoferin.

52. Composition according to Claim 46, characterized in that the receptor antagonist of TNF-α and the inhibitors of the release and/or synthesis of TNF-α are chosen from lisophyline, A802715, sulphasalazine.

53. Composition according to any one of Claims 18 to 52, characterized in that the compound reducing the synthesis, release and/or activity of at least one inflammation mediator is in a quantity representing from 0.001% to 5% of the total weight of the composition and preferably in a quantity representing from 0.01% to 2% of the total weight of the composition.

54. Pharmaceutical composition according to any one of Claims 18 to 52, characterized in that the compound reducing the synthesis, release and/or activity of at least one inflammation mediator is in a quantity representing from 0.001% to 10% of the total weight of the composition and preferably in a quantity representing from 0.01% to 5% of the total weight of the composition.

55. Pharmaceutical composition according to any one of Claims 18 to 52 and 54, intended to treat disorders of the central nervous system, respiratory disorders, allergic syndromes, inflammation, pain, gastrointestinal disorders, skin disorders, fibrosis, collagen maturation disorders, cardiovascular disorders, vasospastic disorders, immunological disorders and/or disorders of the urinary tract.

56. Cosmetic composition according to any one of Claims 18 to 53, intended to prevent and/or combat skin irritations and/or erythemas and/or dysaesthetic sensations and/or sensations of inflammation of the skin and/or the mucous membranes.

57. Pharmaceutical composition according to any one of Claims 18 to 52 or 54 or 55, intended to prevent and/or combat dartres and/or pruritus of the skin and/or the mucous membranes.

58. Composition according to any one of Claims 20 to 57, characterized in that it comprises, in addition, in a cosmetically or pharmaceutically acceptable medium, at least one product with an irritant effect.

59. Composition according to any one of Claims 18 to 58, characterized in that the extract of cells of at least one plant of the Iridaceae family is as defined in Claims 1 to 10.

60. Cosmetic composition according to any one of Claims 18 to 53 or 56 or 58 or 59, characterized in that the said Iridaceae extract is used in a quantity representing from 0.001% to 20% of the total weight of the composition and preferably in a quantity representing from 0.1% to 10% of the total weight of the composition.

61. Pharmaceutical composition according to any one of Claims 18 to 52 or 54 or 55 or 57 to 59, characterized in that the said Iridaceae extract is used in a quantity representing from 0.1% to 30% of the total weight of the composition and preferably in a quantity representing from 0.5% to 20% of the total weight of the composition.

62. Process of non-therapeutic cosmetic treatment, characterized in that a cosmetic composition as defined in any one of Claims 18 to 53 or 56 or 58 to 60 is applied to the skin, the hair and/or the mucous membranes.

## Patentansprüche

1. Verfahren zur nicht therapeutischen, kosmetischen Behandlung von Störungen, die mit einer überhöhten Synthese und/oder Freisetzung von CGRP und/oder der Substanz P verbunden sind,
dadurch gekennzeichnet, daß
eine kosmetische Zusammensetzung verwendet wird, die mindestens einen Extrakt von Zellen mindestens einer Pflanze aus der Familie der Iridaceae enthält.

2. Verfahren zur nicht therapeutischen, kosmetischen Behandlung nach Anspruch 1 zur Behandlung von empfindlicher Haut.

3. Verfahren zur nicht therapeutischen, kosmetischen Behandlung nach Anspruch 1 zur Vorbeugung und/oder Bekämpfung von Hautreizungen und/oder Erythemen und/oder dysästhesischen Empfindungen und/oder Erwärmen der Haut und/oder der Schleimhäute.

4. Verfahren zur nicht therapeutischen, kosmetischen Behandlung nach einem der vorhergehenden Ansprüche, dadurch gekennzeichnet, daß der Extrakt aus Zellen hergestellt wird, die von einer Iridacea einer Gattung stammen, die unter Romulea, Crocus, Iris, Gladiolus, Sisyrinchium und Hermodactylus ausgewählt ist.

5. Verfahren zur nicht therapeutischen, kosmetischen Behandlung nach dem vorhergehenden Anspruch, dadurch gekennzeichnet, daß der Extrakt aus Zellen hergestellt wird, die von einer Iridacea der Gattung Iris stammen.

6. Verfahren zur nicht therapeutischen, kosmetischen Behandlung nach dem vorhergehenden Anspruch, dadurch gekennzeichnet, daß der Extrakt aus Zellen hergestellt wird, die von Iris pallida stammen.

7. Verfahren zur nicht therapeutischen, kosmetischen Behandlung nach einem der vorhergehenden Ansprüche, dadurch gekennzeichnet, daß der Extrakt aus Zellen hergestellt wird, die von einem Pflanzenmaterial aus Invitro-Kultur stammen.

8. Verfahren zur nicht therapeutischen, kosmetischen Behandlung nach einem der vorhergehenden Ansprüche, dadurch gekennzeichnet, daß der Extrakt aus undifferenzierten Zellen hergestellt wird.

9. Verwendung mindestens eines Extrakts von Zellen mindestens einer Pflanze aus der Familie der Iridaceae zur Herstellung einer pharmazeutischen Zusammensetzung zur Behandlung von Krankheiten, die mit CGRP-Antagonisten und/oder Antagonisten der Substanz P behandelt werden können, mit Ausnahme von Atemwegserkrankungen.

10. Verwendung mindestens eines Extrakts von Zellen mindestens einer Pflanze aus der Familie der Iridaceae zur Herstellung einer pharmazeutischen Zusammensetzung, die zur Behandlung von Störungen verwendet werden soll, die mit einer überhöhten Synthese und/oder Freisetzung von CGRP und/oder der Substanz P verbunden sind, mit Ausnahme von Atemwegserkrankungen.

11. Verwendung mindestens eines Extrakts von Zellen mindestens einer Iridacea zur Herstellung einer pharmazeutischen Zusammensetzung zur Behandlung von Störungen des Zentralnervensystems, allergischen Syndromen, Entzündungen, Schmerzen, Erkrankungen des Magen-Darmtrakts, Hautstörungen, Fibrosen, Störungen der Kollagenbildung, kardiovaskulären Störungen, vasospastischen Störungen, immunologischen Störungen und/oder Störungen des Harntrakts.

12. Verwendung mindestens eines Extrakts von Zellen mindestens einer Iridacea zur Herstellung einer pharmazeutischen Zusammensetzung zur Vorbeugung und/oder Bekämpfung von Flechten und/oder Juckreiz der Haut und/oder der Schleimhäute.

13. Verwendung nach einem der Ansprüche 9 bis 12, dadurch gekennzeichnet, daß der Extrakt aus Zellen hergestellt wird, die von einer Iridacea einer Gattung stammen, die unter Romulea, Crocus, Iris, Gladiolus, Sisyrinchium und Hermodactylus ausgewählt ist.

14. Verwendung nach dem vorhergehenden Anspruch, dadurch gekennzeichnet, daß der Extrakt aus Zellen hergestellt wird, die von einer Iridaceae der Gattung Iris stammen.

15. Verwendung nach dem vorhergehenden Anspruch, dadurch gekennzeichnet, daß der Extrakt aus Zellen hergestellt wird, die von Iris pallida stammen.

16. Verwendung nach einem der vorhergehenden Ansprüche, dadurch gekennzeichnet, daß der Extrakt aus Zellen hergestellt wird, die von Pflanzenmaterial aus In-vitro-Kultur stammen.

17. Verwendung nach einem der vorhergehenden Ansprüche, dadurch gekennzeichnet, daß der Extrakt aus undifferenzierten Zellen hergestellt wird.

18. Kosmetische oder pharmazeutische Zusammensetzung, dadurch gekennzeichnet, daß sie in einem kosmetisch oder pharmazeutisch akzeptablen Medium einen Extrakt von Zellen mindestens einer Pflanze der Familie der Iridaceae und mindestens ein Produkt mit reizender Wirkung, mit Ausnahme von Ascorbinsäure, enthält.

19. Zusammensetzung nach Anspruch 18, dadurch gekennzeichnet, daß das Produkt mit reizender Wirkung unter den ionischen oder nichtionischen grenzflächenaktiven Stoffen, Konservierungsmitteln, organischen Lösungsmitteln oder Wirkstoffen, wie α-Hydroxysäuren, β-Hydroxysäuren, α-Ketosäuren, β-Ketosäuren, Retinoiden, Anthralinen, Anthranoiden, Peroxiden, Minoxidil, Lithiumsalzen, Antimetaboliten, Vitamin D und seinen Derivaten, Färbemitteln oder Farbstoffen für das Haar, parfümierenden alkoholischen Lösungen, Antitranspirantien, Haarentfernungsmitteln, Wirkstoffen für permanente Verformungen und depigmentierenden Wirkstoffen ausgewählt ist.

20. Kosmetische oder pharmazeutische Zusammensetzung, dadurch gekennzeichnet, daß sie in einem kosmetisch oder pharmazeutisch akzeptablen Medium einen Extrakt von Zellen mindestens einer Pflanze der Familie der Iridaceae und eine Verbindung enthält, die die Synthese, die Freisetzung und/oder die Aktivität mindestens eines Entzündungsmediators vermindert, wobei Kojisäure ausgenommen ist.

21. Zusammensetzung nach Anspruch 20, dadurch gekennzeichnet, daß die Verbindung, die die Synthese, die Freisetzung und/oder die Aktivität mindestens eines Entzündungsmediators vermindert, eine Verbindung ist, die die Synthese, die Freisetzung und/oder die Aktivität mindestens eines Entzündungsmediators der Haut vermindert.

22. Zusammensetzung nach einem der Ansprüche 20 oder 21, dadurch gekennzeichnet, daß die Verbindung, die die Synthese, die Freisetzung und/oder die Aktivität mindestens eines Entzündungsmediators vermindert, unter den Antagonisten der Substanz P und/oder den Antagonisten von CGRP, den Inhibitoren der NO-Synthase, den Antagonisten von Bradykinin, den Antagonisten von Cytokinen, den Antagonisten von Histamin, den Antagonisten des Tumornecrose-Ffaktors vom α-Typ (TNF α) ausgewählt ist.

23. Zusammensetzung nach Anspruch 22, dadurch gekennzeichnet, daß die Antagonisten rezeptorielle Antagonisten sind.

24. Zusammensetzung nach einem der Ansprüche 22 oder 23, dadurch gekennzeichnet, daß der Antagonist der Substanz P unter den Substanzen, die eine Verminderung der Extravasation von Plasma durch die Gefäßwand gewährleisten, die durch Capsaicin oder eine antidrome Nervenstimulierung hervorgerufen wird, und den Substanzen ausgewählt ist, die eine Inhibierung der durch die Verabreichung der Substanz P bewirkte Kontraktion der glatten Muskulatur hervorrufen.

25. Zusammensetzung nach einem der Ansprüche 22 bis 24, dadurch gekennzeichnet, daß der Antagonist der Substanz P unter den Peptiden, den Verbindungen, die mindestens einen Heterocyclus enthalten, den Stickstoffverbindungen, die mindestens einen Benzolring aufweisen, den Salzen von einwertigen, zweiwertigen und dreiwertigen Kationen, den Thermalwässern und deren Gemischen ausgewählt ist.

26. Zusammensetzung nach Anspruch 25, dadurch gekennzeichnet, daß das Peptid das Sendide oder das Spantide II ist.

27. Zusammensetzung nach Anspruch 25, dadurch gekennzeichnet, daß die Verbindung, die mindestens einen Heterocyclus aufweist, eine heterocyclische Stickstoff-, Sauerstoff- oder Schwefelverbindung ist, die unter den Derivaten von 2-Tricyclyl-2-aminoethan, den Derivaten von Spirolactam, den Derivaten von Chinuclidin, den azacyclischen Derivaten, den Derivaten von Aminopyrrolidin, den Derivaten von Piperidin, den Aminoazaheterocyclen, den Derivaten von Isoindol, den Derivaten von Furan, den Derivaten von Benzofuran, den Derivaten von Thiophen und den Derivaten von Benzothiophen und insbesondere den Tetraazolyl-benzofurancarboxamiden oder den Tetrazolyl-benzothiophencarboxamiden ausgewählt ist.

28. Zusammensetzung nach Anspruch 25, dadurch gekennzeichnet, daß das Salz unter den Chloriden, Acetaten, Carbonaten, Hydrogencarbonaten, Salicylaten, Boraten, Nitraten, Hydroxiden, Sulfaten, Persulfaten, Glycerophosphaten, Salzen von α-Hydroxysäuren, Salzen von Fruchtsäuren, Salzen von Aminosäuren und Salzen von Fettsäuren von Strontium, Magnesium, Lanthaniden mit einer Ordnungszahl im Bereich von 57 bis 71, Cobalt, Nickel, Mangan, Barium, Yttrium, Kupfer, Zinn, Rubidium, Lithium und Zink ausgewählt ist.

29. Zusammensetzung nach Anspruch 28, dadurch gekennzeichnet, daß das Salz unter den Strontiumsalzen ausgewählt ist.

30. Zusammensetzung nach einem der Ansprüche 28 oder 29, dadurch gekennzeichnet, daß das Salz das Strontiumchlorid oder Strontiumnitrat ist.

31. Zusammensetzung nach Anspruch 25, dadurch gekennzeichnet, daß das Thermalwasser ein Wasser ist, das aus einer Quelle aus den Becken von Vichy stammt.

32. Zusammensetzung nach Anspruch 31, dadurch gekennzeichnet, daß das Thermalwasser aus den Quellen Célestins, Chomel, Grande-Ville, Hôpital, Lucas und Parc stammt.

33. Zusammensetzung nach einem der Ansprüche 31 oder 32, dadurch gekennzeichnet, daß das Thermalwasser aus der Lucas-Quelle stammt.

34. Zusammensetzung nach einem der Ansprüche 22 oder 23, dadurch gekennzeichnet, daß der CGRP-Antagonist unter den Substanzen, die eine Verminderung der durch Capsaicin oder eine antidrome elektrische Stimulierung (an einen afferenten Nerv angelegt) hervorgerufene Vasodilatation und/oder eine Inhibierung der Freisetzung von CGRP durch die sensitiven Nervenfasern gewährleisten, und den Substanzen ausgewählt ist, die eine Inhibierung der durch CGRP hervorgerufenen Kontraktion der glatten Muskulatur des Samenleiters hervorrufen.

35. Zusammensetzung nach Anspruch 34, dadurch gekennzeichnet, daß der Antagonist von CGRP unter CGRP 8-37 (Aminosäuresequenz 8 bis 37 des N-terminalen Teils von CGRP) oder den anti-CGRP-Antikörpern ausgewählt ist.

36. Zusammensetzung nach Anspruch 22, dadurch gekennzeichnet, daß der Inhibitor der NO-Synthase unter den Substanzen ausgewählt ist, durch die die Synthese von Stickstoffmonoxid (NO) in situ am Menschen teilweise oder sogar vollständig inhibiert werden kann.

37. Zusammensetzung nach Anspruch 36, dadurch gekennzeichnet, daß der Inhibitor der NO-Synthase unter den Verbindungen, die die Synthese der NO-Synthase inhibieren und/oder den Katabolismus der NO-Synthase beschleunigen, den Verbindungen, die die NO-Synthase neutralisieren, oder den Verbindungen ausgewählt ist, die wirken, indem das durch die NO-Synthase übermittelte Signal vermindert wird.

38. Zusammensetzung nach einem der Ansprüche 36 oder 37, dadurch gekennzeichnet, daß der Inhibitor der NO-Synthase unter den synthetischen oder natürlichen Peptiden, die gegebenenfalls modifiziert sind, chemischen, synthetischen oder natürlichen Molekülen, Antisens-Nucleinsäuren, Ribozymen und anti-NO-Synthase-Antikörpern ausgewählt ist.

39. Zusammensetzung nach einem der Ansprüche 36 bis 38, dadurch gekennzeichnet, daß der Inhibitor der NO-Synthase ausgewählt ist unter N^{G}-Monomethyl-L-arginin (L-NMMA), N^{G}-Nitro-L-arginin, N^{G}-Nitro-L-arginin-methylester, Diphenyleniodoniumchlorid, 7-Nitroindazol, N(5)-(1-iminoethyl)-L-ornithin, N^{G},N^{G}-Dimethyl-L-arginin, N^{G},N^{G}-Dimethyl-arginin, 2-(4-Carboxyphenyl)-4,4,5,5-tetra-methylimidazolin-1-oxy-3-oxid, Ebselen, Aminoguanidin, Canavanin und dem α-Melanocyten-stimulierenden-Hormon.

40. Zusammensetzung nach einem der Ansprüche 36 bis 38, dadurch gekennzeichnet, daß der Inhibitor der NO-Synthase das N^{G}-Monomethyl-L-arginin oder das α-Melanocyten-stimulierende-Hormon ist.

41. Zusammensetzung nach einem der Ansprüche 22 oder 23, dadurch gekennzeichnet, daß der Bradykinin-Antagonist ausgewählt ist unter den Verbindungen, die die Synthese von Bradykinin inhibieren und/oder den Katabolismus von Bradykinin beschleunigen, Verbindungen, die das Bradykinin neutralisieren, Verbindungen, die die Bradykinin-Rezeptoren blockieren, wie beispielsweise die Verbindungen, die mit den Wirkungen des Bradykinins durch ihre Fixierung an den Badykinin-Rezeptor (B1 oder B2) interferieren, Verbindungen, die die Synthese von Bradykinin-Rezeptoren inhibieren, oder Verbindungen, die wirken, indem das von Bradykinin übermittelte Signal vermindert wird.

42. Zusammensetzung nach Anspruch 41, dadurch gekennzeichnet, daß der Bradykinin-Antagonist unter synthetischen oder natürlichen Peptiden, die gegebenenfalls modifiziert sind, chemischen, synthetischen oder natürlichen Molekülen, Antisens-Nucleinsäuren, Ribozymen, anti-Bradykinin-Antikörpern, löslichen Bradykinin-Rezeptoren, anti-Bradykinin-Rezeptor-Antikörpern oder Antagonisten der Bradykinin-Rezeptoren ausgewählt ist.

43. Zusammensetzung nach einem der Ansprüche 41 oder 42, dadurch gekennzeichnet, daß der Bradykinin-Antagonist unter den Verbindungen ausgewählt ist, die mit den Wirkungen des Bradykinins durch ihre Fixierung an den Bradykinin-Rezeptor (B1 oder B2) und vorzugsweise den Rezeptor B2 interferieren.

44. Zusammensetzung nach einem der Ansprüche 41 bis 43, dadurch gekennzeichnet, daß der Bradykinin-Antagonist ausgewählt ist unter D-Arg, [Hyp3, D-Phe7]-bradykinin (NPC567), [Thi5,8, D-Phe7]-bradykinin, D-Arg, [Hyp3, Thi5,8, D-Phe7]-bradykinin, N-α-Adamantanacetyl-D-Arg, [Hyp3, Thi5,8, D-Phe7]-bradykinin, des-Arg9, [Leu8]-bradykinin, P-Guanidobenzoyl, [Hyp3, Thi5, D-Tic7, Oic8]-bradykinin (S 16118), D-Arg, [Hyp3, Thi5, D-Tic7, Oic8]-bradykinin (HOE 140)und D-Arg, [Hyp3, D-Hype(trans-propyl)7, Oic8]-bradykinin (NPC 13731).

45. Zusammensetzung nach einem der Ansprüche 41 bis 44, dadurch gekennzeichnet, daß der Bradykinin-Antagonist das D-Arg-[Hype, Thi5, D-Tic7, Oic8]-bradykinin (HOE 140) ist.

46. Zusammensetzung nach einem der Ansprüche 22 oder 23, dadurch gekennzeichnet, daß der Antagonist von Histamin, Cytokinen oder TNF-α eine Substanz ist, die unter den rezeptoriellen Antagonisten von Histamin, Cytokinen oder TNF-α oder unter den Antagonisten der Freisetzung und/oder Synthese von Histamin, Cytokinen oder TNF-α ausgewählt ist.

47. Zusammensetzung nach Anspruch 46, dadurch gekennzeichnet, daß die unter den rezeptoriellen Antagonisten von Histamin, Cytokinen oder TNF-α ausgewählte Substanz eine Substanz ist, die eine selektive Affinität für die spezifischen Rezeptoren dieser Verbindungen aufweist oder eine Inhibierung der durch die Verabreichung von Histamin hervorgerufene Kontraktion der glatten Muskulatur gewährleistet oder eine Inhibierung der durch die Cytokine hervorgerufene Haftung von Makrophagen an Endothelium-Zellen gewährleistet oder eine Inhibierung der durch Cytokine an Neurophilen hervorgerufene Freisetzung von Superoxidanionen gewährleistet oder eine Inhibierung der durch TNF-α hervorgerufenen Haftung von Makrophagen an Endothelium-Zellen gewährleistet oder eine Inhibierung der durch TNF-α an Neurophilen hervorgerufenen Freisetzung von Superoxidanionen gewährleistet oder eine Inhibierung der mitogenen Wirkung des TNF-α auf Fibroblasten der Dermis gewährleistet.

48. Zusammensetzung nach einem der Ansprüche 46 oder 47, dadurch gekennzeichnet, daß der rezeptorielle Histamin-Antagonist unter den Derivaten von Diethylendiamin wie Cinnarizin, Cyclizin; den Derivaten von Aminopropan, wie Dexchlorrpheniramin, Tripolidin; den Derivaten von Phenothiazin, wie Promethazin, Alimemazin; Cetirezin HCl, Ebastin, Loratadin und Setastin HCl ausgewählt ist.

49. Zusammensetzung nach Anspruch 46, dadurch gekennzeichnet, daß der Antagonist der Freisetzung von Histamin unter den heterocyclischen Sauerstoff- oder Schwefelverbindungen, wie beispielsweise Furanderivaten, Benzofuranderivaten, Thiophenderivaten und Benzothiophenderivaten, die gegebenenfalls Stickstoffsubstituenten enthalten, und vorzugsweise unter den Alkoxy- und/oder Aryloxytetrazolylbenzofurancarboxamiden oder den Alkoxy- und/oder Aryloxy-tetrazolylbenzothiophencarboxamiden ausgewählt ist.

50. Zusammensetzung nach einem der Ansprüche 46 bis 49, dadurch gekennzeichnet, daß der Antagonist der Freisetzung von Histamin unter 5-Methoxy-3-phenoxy-N-1H-tetrazol-5-yl-benzothiophen-2-carboxamid, 5-Methoxy-3-(1-methylethoxy)-N-1H-tetrazol-5-yl-benzothiophen-2-carboxamid, 6-Methoxy-3-(1-methylethoxy)-N-1H-tetrazol-5-yl-benzothiophen-2-carboxamid, 5-Methoxy-3-(1-methyl-ethyl)-N-1H-tetrazol-5-yl-benzothiophen-2-carboxamid, 3-Benzyloxy-5-methoxy-N-1H-tetrazol-5-yl-benzothiophen-2-carboxamid und 5-Methoxy-3-phenoxy-N-1H-tetrazol-5-yl-benzothiophen-2-carboxamid ausgewählt ist.

51. Zusammensetzung nach Anspruch 46, dadurch gekennzeichnet, daß der Antagonist von Cytokinen unter den Antagonisten der Freisetzung von Interleukin 1, wie Auranofin oder SKF-105809, oder den Antagonisten der Synthese von Interleukin 1, wie Lactoferrin, ausgewählt ist.

52. Zusammensetzung nach Anspruch 46, dadurch gekennzeichnet, daß der rezeptorielle Antagonist von TNF-α und die Inhibitoren der Freisetzung und/oder Synthese von TNF-α unter Lisophylin, A802715 und Sulfasalazin ausgewählt sind.

53. Kosmetische Zusammensetzung nach einem der Ansprüche 18 bis 52, dadurch gekennzeichnet, daß die Verbindung, die die Synthese, Freisetzung und/oder Aktivität mindestens eines Entzündungsmediators vermindert, in einem Mengenanteil im Bereich von 0,001 bis 5 % des Gesamtgewichts der Zusammensetzung und vorzugsweise in einem Mengenanteil im Bereich von 0,01 bis 2 % des Gesamtgewichts der Zusammensetzung vorliegt.

54. Pharmazeutische Zusammensetzung nach einem der Ansprüche 18 bis 52, dadurch gekennzeichnet, daß die Verbindung, die die Synthese, Freisetzung und/oder Aktivität mindestens eines Entzündungsmediators vermindert, in einem Mengenanteil im Bereich von 0,001 bis 10 % des Gesamtgewichts der Zusammensetzung und vorzugsweise in einem Mengenanteil im Bereich von 0,01 bis 5 % des Gesamtgewichts der Zusammensetzung vorliegt.

55. Pharmazeutische Zusammensetzung nach einem der Ansprüche 18 bis 52 und 54 zur Behandlung von Störungen des Zentralnervensystems, Atemwegsstörungen, allergischen Syndromen, Entzündungen, Schmerzen, Störungen des Magen-Darmtrakts, Hautstörungen, Fibrosen, Störungen der Kollagenbildung, kardiovaskulären Störungen, vasospastischen Störungen, immunologischen Störungen und/oder Störungen des Harntrakts.

56. Kosmetische Zusammensetzung nach einem der Ansprüche 18 bis 53 zur Vorbeugung und/oder Bekämpfung von Hautreizungen und/oder Erythemen und/oder dysästhesischen Empfindungen und/oder Hitzegefühlen der Haut und/oder der Schleimhäute.

57. Pharmazeutische Zusammensetzung nach einem der Ansprüche 18 bis 52 oder 54 oder 55 zur Vorbeugung und/oder Bekämpfung von Flechten und/oder Juckreiz der Haut und/oder der Schleimhäute.

58. Zusammensetzung nach einem der Ansprüche 20 bis 57, dadurch gekennzeichnet, daß sie ferner in einem kosmetisch oder pharmazeutisch akzeptablen Medium mindestens ein Produkt mit reizender Wirkung enthält.

59. Zusammensetzung nach einem der Ansprüche 18 bis 58, dadurch gekennzeichnet, daß der Extrakt von Zellen mindestens einer Pflanze aus der Familie der Iridaceae ein Extrakt nach einem der Ansprüche 1 bis 10 ist.

60. Kosmetische Zusammensetzung nach einem der Ansprüche 18 bis 53 oder 56 oder 58 oder 59, dadurch gekennzeichnet, daß der Extrakt von Iridaceae in einem Mengenanteil im Bereich von 0,001 bis 20 % des Gesamtgewichts der Zusammensetzung und vorzugsweise in einem Mengenanteil von 0,1 bis 10 % des Gesamtgewichts der Zusammensetzung verwendet wird.

61. Pharmazeutische Zusammensetzung nach einem der Ansprüche 18 bis 52 oder 54 oder 55 oder 57 bis 59, dadurch gekennzeichnet, daß der Extrakt von Iridaceae in einem Mengenanteil von 0,1 bis 30 % des Gesamtgewichts der Zusammensetzung und vorzugsweise in einem Mengenanteil im Bereich von 0,5 bis 20 % des Gesamtgewichts der Zusammensetzung verwendet wird.

62. Verfahren zur nicht therapeutischen, kosmetischen Behandlung, dadurch gekennzeichnet, daß auf die Haut, die Haare und/oder die Schleimhäute eine kosmetische Zusammensetzung nach einem der Ansprüche 18 bis 53 oder 56 oder 58 oder 60 aufgetragen wird.
